# FASCICULE DE BREVET EUROPEEN

(11) **EP 0 643 776 B1**
(45) Date de publication et mention de la délivrance du brevet: **04.09.1996**
(21) Numéro de dépôt: 93913096.9
(22) Date de dépôt: 04.06.1993
(51) Int. Cl.: C12Q 1/68, C07K 14/195

(54) **SEQUENCES NUCLEOTIDIQUES ISSUES DE GENES CODANT DES PECTATE-LYASES, ET LEURS UTILISATIONS NOTAMMENT POUR LA DETECTION DE BACTERIES DU GENRE ERWINIA**
NUKLEOTIDISCHE SEQUENTIEN VON GENEN DIE FÜR PECTATE-LIASEN KODIEREN UND DESSEN VERWENDUNG FÜR DEN NACHWEIS VON BAKTERIEN DER GATTUNG ERWINIA
NUCLEOTIDIQUE SEQUENCES OBTAINED FROM GENES CODING PECTATE-LYASES, AND UTILIZATIONS THEREOF PARTICULARLY FOR THE DETECTION OF BACTERIA OF THE GENUS ERWINIA

(30) Priorité: 05.06.1992 FR 9206888
(43) Date de publication de la demande: 22.03.1995
(73) Titulaire: INSTITUT NATIONAL DE LA RECHERCHE AGRONOMIQUE, F-75341 Paris Cédex 07 (FR); INSTITUT NATIONAL AGRONOMIQUE PARIS-GRIGNON ( INA-PG), F-75231 Paris Cédex 05 (FR)
(72) Inventeur: DARRASSE, Armelle, F-40350 Pouillon (FR); KOTOUJANSKY, Alain, F-75013 Paris (FR); BERTHEAU, Yves, F-75013 Paris (FR)
(74) Mandataire: Grosset-Fournier, Chantal Catherine
(86) Numéro de dépôt international: FR9300540
(87) Numéro de publication internationale: WO9325708

(56) Documents cités:
- EP-A- 0 409 159
- DE-A- 3 419 382
- BIOLOGICAL ABSTRACTS, vol. 90, no. 11, 1990, Philadelphia, PA (US); AN 127177
- BIOCHEMICAL & BIOPHYSICAL RESEARCH COMMUNICATION, vol. 168, no. 2, 1990; T. NISHIDA et al., pp. 801-808
- GENE, vol. 62, 1988, Amsterdam (NL); S.P. LEI et al., pp. 159-164
- DERWENT WPIL, Week 9240, Derwent Publications Ltd., London (GB); AN 92-321179
- PLANT & SOIL, vol. 125, 1990; pp. 285-287

## Description

La présente invention a pour objet des séquences nucléotidiques issues de gènes codant des pectacte-lyases, et utilisables, notamment en tant que sondes et amorces, pour la détection de bactéries pectinolytiques du genre *Erwinia* chez des hôtes susceptibles d'être infectés par ces dernières, ou dans tout milieu où ces bactéries sont susceptibles de survivre.

Une étude effectuée en 1988 indiquait que les pertes mondiales dues aux maladies des plantes s'élevaient couramment à 60 milliards de dollars par an. C'est dire l'importance que représente la détection rapide et spécifique des agents phytopathogènes, qui conditionne, entre autres, les choix des produits phytosanitaires, de leur dosage et du moment de leur application.

Les mesures prophylactiques, nécessitant donc une détection rapide, sensible et fiable, constituent encore de nos jours un des moyens les plus efficaces et les moins coûteux, tant au niveau financier qu'à celui de l'environnement, de lutte contre les maladies, et en particulier les bactérioses contre lesquels peu de moyens de lutte sont disponibles, l'utilisation d'antibiotiques étant prohibée dans la majorité des pays. Ces mesures prophylactiques consistent essentiellement en l'utilisation de plantes, boutures ou semences (pris au sens large du terme) saines, après avoir détruit ou refusé les lots contaminés, en stérilisation ou tout autre procédé de désinfection de l'ensemble des matériaux susceptibles d'être en contact avec les plantes et les semences.

Le dépistage des pathologies d'origine bactérienne chez les plantes n'a cependant pas été satisfaisant jusqu'à présent, et des pertes importantes de plantes mono et dicotylédones dans les champs, dans les serres ou durant leur stockage sont fréquemment observées.

Une des causes principales de ce problème est l'utilisation de semences et boutures apparemment saines, mais qui sont en réalité porteuses de bactéries susceptibles d'être à l'origine du développement futur de pathologies chez les plantes obtenues à partir de ces semences. On parlera alors de bactéries en phase latente d'infection.

De nombreuses pathologies de plantes sont caractérisées par une phase latente d'infection. Durant cette phase, le niveau d'infection est généralement inférieur à celui qui pourrait être détecté par inspection visuelle (absence de symptômes apparents) ou par les techniques courantes disponibles actuellement (isolement et caractérisation microbiologique, immunologie...).

Une autre source importante de contamination des plantes viendrait également de l'environnement naturel ou artificiel, notamment des sols et de l'eau.

Il faut noter également que l'isolement et la caractérisation microbienne sont en général des techniques longues et laborieuses, susceptibles d'être d'un coût trop élevé pour envisager de les appliquer à grande échelle, tandis que les méthodes immunologiques ne présentent pas toujours la spécificité et la sensibilité désirées.

Les bactéries pectinolytiques du genre *Erwinia* (encore désignées ci-après erwinias) sont responsables d'un grand nombre de ce type de pathologies, dont l'un des symptômes est la macération des tissus chez un grand nombre de plantes, boutures et semences (terme pris au sens large). Ces bactéries sont notamment capables d'infecter la pomme de terre, le maïs, la tomate, la betterave à sucre, le chou, l'endive, etc..., ainsi que les plantes ornementales de serres, telles que les plantes ornementales, et d'une manière générale toute plante couramment échangée sur le marché entre les pays européens et des pays tiers.

Parmi ces erwinias pectinolytiques, on peut citer principalement d'une part *Erwinia carotovora,* et plus particulièrement *Erwinia carotovora* subsp. *carotovora (Ecc), Erwinia carotovora* subsp. *atroseptica (Eca), Erwinia carotovora* subsp. *odorifera (Eco),* et *Erwinia carotovora* subsp. *wasabiae (Ecw),* et d'autre part, *Erwinia chrysanthemi (Ech).*

Ces bactéries produisent plusieurs enzymes extracellulaires capables d'attaquer les composants de la paroi cellulaire; pectinases, cellulases, hémicellulases et protéases (pour une revue détaillée, voir l'article de A. Kotoujansky paru dans Annu. Rev. Phytopathol. 1987, 25: 405-30).

Parmi ces enzymes, les pectate-lyases (encore désignées par polygalacturonate-lyases, pectate-transéliminases, polygalacturonatetranséliminases, poly(1,4-α-D-galacturonide)lyase, et EC 4.2.2.2) participeraient au mécanisme de cette macération des plantes, sans pour autant être les seuls agents responsables de ce phénomène, et ne seraient pas toutes nécessaires à la virulence de ces bactéries.

Par pectate-lyases on entend toute enzyme capable, par le biais d'une réaction de β-élimination, de couper une liaison α 1->4 O glycosidique présente entre deux résidus galacturoniques.

Ces pectate-lyases (encore désignées ci-après par PL) sont principalement codées par des gènes *pel* nommés *pelY* (Manulis *et al.,* 1988), *pelB* (Lei *et al.,* 1987), *pelA* (Lei et *al.,* 1988), *pell* (Ito *et al.,* 1988), *pel153* (Trollinger *et al.,* 1989), *pelA, pelC, pelE* et *pelD* (Tamaki *et al.,* 1988), *pelA, pelD* et *pelE* (Van Gijsegem, 1989), *pelB* (Schoedel et Collmer, 1986), *pelA* (Favey *et al.,* 1992) et *pelB* (Hinton *et al.,* 1989).

Ces gènes ont eux-mêmes été regroupés en trois familles en fonction de leurs homologies de séquences (Hinton *et al.,* 1989). Il a en effet été déterminé que la famille *pelA, D, E* est présente chez *Ech,* et la famille *pelB, C, I, X,* est présente chez *Eca, Ecc* et *Ech,* et que la famille *pel153, Y* est présente chez certaines souches *d'Erwinia carotovora.*

Il convient cependant de noter que ces gènes ne sont pas spécifiques des erwinias pectinolytiques. En effet, d'autres bactéries, telles que *Yersinia pseudotuberculosis (Yps), Klebsiella pneumoniae* et *Pseudomonas* sp., sont capables de produire des PL. La famille *pel153, Y* aurait été mise en évidence chez *Yps;* une partie du gène *pel153* serait effectivement commune à *Eca* et *Yps* (Trollinger *et al.,* 1989).

La présence de séquences conservées tant au niveau des séquences nucléotidiques que protéiques correspondant à ces PL, serait à l'origine de l'apparition de réactions croisées lors de la mise en oeuvre de méthodes de détection des erwinias, que ces méthodes procèdent par hybridation entre acides nucléiques, ou par réaction immunologique à l'aide d'anticorps anti-PL.

C'est principalement cette possibilité de réactions croisées qui est à l'origine du fait qu'aucune méthode de diagnostic des pathologies causées par ces erwinias pectinolytiques, procédant par l'intermédiaire de séquences nucléotidiques correspondant à ces PL, n'a été envisagée.

Quant aux méthodes de diagnostic immunologiques de ces pathologies par détection de ces PL, outre ce problème de réactions croisées (donnant lieu à l'apparition de faux positifs ou de faux négatifs), elles présentent également l'inconvénient de ne pas permettre le diagnostic précoce des pathologies en question, compte tenu du fait que la présence de ces PL dans le milieu étudié est concomitante à l'apparition des premiers symptômes de ces pathologies.

Le document Plant and Soil, 125, pages 285-287, 1990, décrit un fragment d'ADN codant une pectate lyase isolée à partir d'une souche de la sous-espèce Erwinia carotova carotova (Ecc).

La présente invention a précisément pour but de mettre à la disposition d'utilisateurs potentiels, des méthodes fiables d'identification et de détection de bactéries pectinolytiques du genre *Erwinia* d'une espèce déterminée, et plus particulièrement, de l'espèce *Erwinia chrysanthemi* et de l'espèce *Erwinia carotovora,* ainsi que de leurs sous-espèces et pathovars.

L'invention a plus précisément pour but de fournir de nouvelles séquences nucléotidiques utilisables en tant que sondes ou amorces pour la mise en oeuvre de ces méthodes, notamment par les techniques procédant par hybridation entre acides nucléiques (ARN et/ou ADN), suivie de l'amplification du nombre de copies de séquences cibles (ou de leurs séquences complémentaires) à détecter, par exemple suivant les techniques d'amplification génique dites PCR (Polymerase Chain Reaction), LCR (Ligase Chain Reaction), Qβ-replicase et 3SR (Self-Sustained Sequence Replication).

L'invention a également pour but l'application des méthodes susmentionnées au diagnostic précoce des pathologies causées par les erwinias pectinolytiques, à savoir des méthodes permettant de faire le diagnostic du risque d'apparition de ces pathologies chez les plantes, ou d'apprécier les risques de contamination des plantes, notamment à partir du sol ou de l'eau.

L'invention a également pour but de mettre à la disposition d'utilisateurs potentiels, des kits pour la mise en oeuvre des méthodes susmentionnées.

L'invention a également pour but l'utilisation des séquences nucléotidiques susmentionnées pour la détection du polymorphisme des gènes codant des pectate-lyases chez les erwinias pectinolytiques, afin d'identifier les espèces, les sous-espèces et pathovars de ces bactéries, mais aussi afin de déterminer l'origine géographique et la spécificité d'hôte des souches de ces espèces, sous-espèces et pathovars, et de fournir des méthodes de diagnostic des pathologies causées spécifiquement par ces espèces, sous-espèces et pathovars, ou en association avec d'autres.

La présente invention sera plus particulièrement illustrée à l'aide des figures suivantes:
- figure 1: séquence nucléotidique du gène *pel153* de la souche EC153 *d'Erwinia carotovora,*
- figure 2: séquence nucléotidique du gène *pelE* de la souche EC16 *d'Erwinia chrysanthemi,*
- figure 3: carte de sites de restriction correspondant aux profils obtenus avec (a) *Sau*3A1, (b) *Hae*II, (c) *Alu*I et (d) *Hpa*II chez *Erwinia carotovora*. La carte est représentée seulement entre les deux amorces dans la région amplifiée du gène *pel.*

La présente invention a pour objet un procédé d'identification et de détection de bactéries pectinolytiques du genre *Erwinia* d'une espèce déterminée, et plus particulièrement de l'espèce *Erwinia chrysanthemi* et de l'espèce *Erwinia carotovora,* dans tout milieu susceptible de contenir de telles bactéries, notamment dans le sol ou l'eau, ou chez un hôte susceptible d'être porteur de telles bactéries, notamment chez les plantes, les boutures, et les semences, ce procédé consistant à rechercher les fragments d'ADN ou d'ARN, spécifiques de l'espèce concernée, codant une pectate-lyase (PL), en mettant en jeu une hybridation moléculaire avec au moins une sonde spécifique, cette hybridation étant précédée par une amplification du nombre de copies des fragments d'ADN susmentionnés à l'aide d'amorces spécifiques.

L'invention concerne plus particulièrement les séquences nucléotidiques correspondant à tout ou partie des gènes qui, au sein du génome des erwinias pectinolytiques, codent des PL, ces séquences nucléotidiques étant utilisables en tant qu'amorces et/ou sondes pour la mise en oeuvre du procédé susmentionné de l'invention.

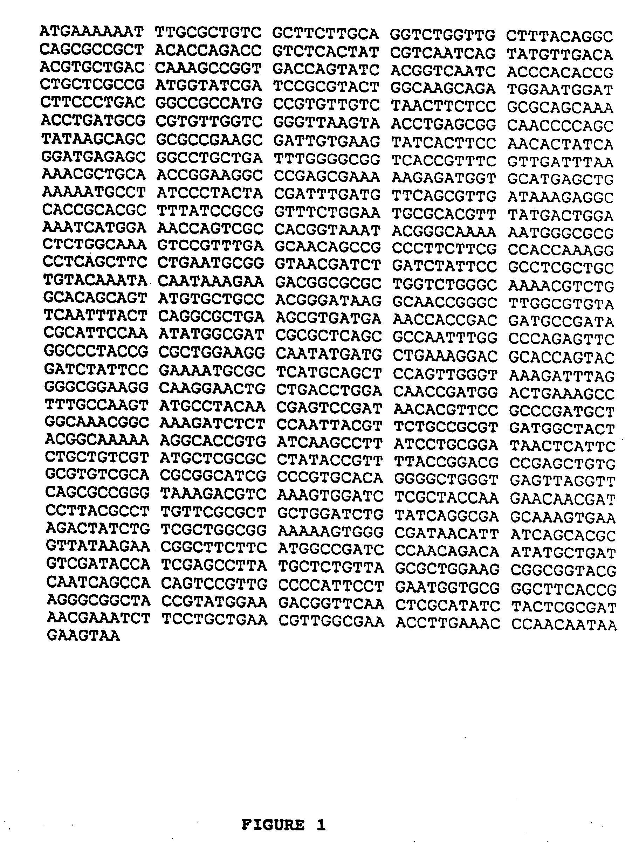

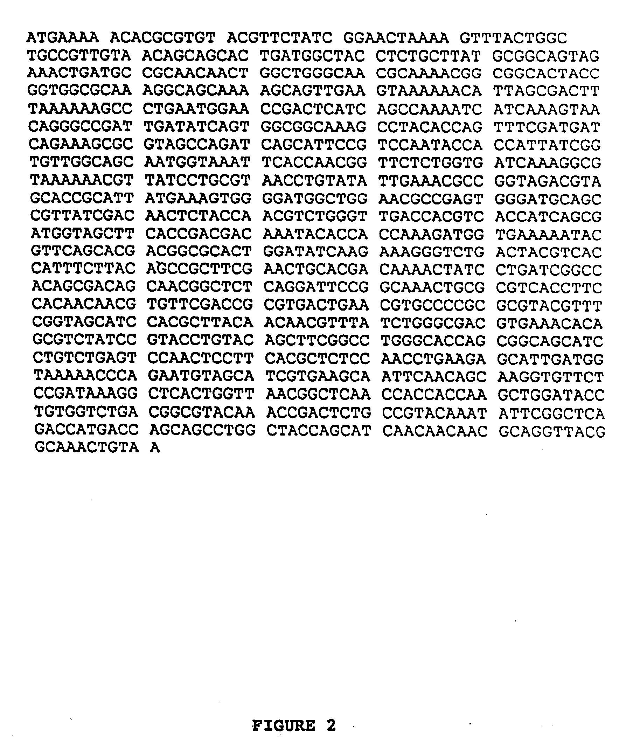

A ce titre l'invention a plus particulièrement pour objet l'utilisation, aux fins susmentionnées, de séquences nucléotidiques correspondant à tout ou partie des phases ouvertes de lecture des gènes codant des PL chez *Erwinia carotovora* et *Erwinia chrysanthemi* (notamment du gène *pel153* représenté sur la figure 1, de la souche EC153 *d'Eca,* du gène *pelA* de la souche 3937 *d'Ech* décrite dans l'article de Favey *et al.* susmentionné, du gène *pelE* de la souche EC16 *d'Ech* représenté sur la figure 2).

Par séquences nucléotidiques correspondant à tout ou partie de gènes qui, au sein des génomes des bactéries pectinolytiques du genre *Erwinia,* codent des pectate-lyases, on entend plus particulièrement:
- soit celles correspondant à l'identique à tout ou partie d'un des brins des séquences d'ADN des gènes codant des PL, ou à tout ou partie des séquences d'ARN messager (ARNm) issues de ces gènes,
- soit celles susceptibles de s'hybrider avec tout ou partie des séquences d'ARNm ou d'un des brins d'ADN susmentionnés. A titre illustratif, les conditions de l'hybridation susmentionnée sont suffisamment stringentes de manière à ce que l'hybridation obtenue résiste à deux lavages successifs d'environ 15 mn chacun dans une solution 3 x SSC (0,5M Nacl, 0,05M citrate de sodium) à une température d'environ 65°C; avantageusement, ces séquences comprennent au moins environ 40% de nucléotides identiques aux séquences d'ADN et d'ARNm susmentionnées.

L'invention a plus particulièrement pour objet toute séquence nucléotidique comprenant tout ou partie de la séquence nucléotidique A suivante: 5'-TTACCGGACGCCGAGCTGTGGCGT-3' ou sa séquence complémentaire, ou une séquence dérivée de la séquence A, notamment par suppression et/ou substitution et/ou addition de nucléotides, et susceptible de s'hybrider avec le fragment délimité par les positions 1231 et 1254 de la séquence représentée sur la figure 1, ou la séquence complémentaire de cette séquence dérivée.

L'invention a également pour objet toute séquence nucléotidique comprenant tout ou partie de la séquence nucléotidique B choisie parmi tout ou partie:
* des enchaînements de nucléotides suivants: 
5'-CAGGAAGATGTCGTTATCGCGAGT-3'
.........C..............
.........T..............
 ou leurs séquences complémentaires,
* ou d'une séquence dérivée de la séquence B, notamment par suppression et/ou substitution et/ou addition de nucléotides, et susceptible de s'hybrider avec la séquence complémentaire du fragment délimité par les positions 1642 et 1665 de la séquence représentée sur la figure 1, ou la séquence complémentaire de cette séquence dérivée.

L'invention vise également toute séquence nucléotidique comprenant tout ou partie de la séquence nucléotidique C choisie parmi tout ou partie:
* des enchaînements de nucléotides suivants: 
5'-GATCAGAAAGCCCGCAGCCAGAT-3'
.....A........T........
...........G..T........
 (les points représentant des nucléotides identiques à ceux de la première ligne) ou leurs séquences complémentaires,
* ou d'une séquence dérivée de la séquence C, notamment par suppression et/ou substitution et/ou addition de nucléotides, et susceptible de s'hybrider avec le fragment délimité par les positions 295 et 317 de la séquence représentée sur la figure 2, ou la séquence complémentaire de cette séquence dérivée.

L'invention concerne également toute séquence nucléotidique comprenant tout ou partie de la séquence nucléotidique D choisie parmi tout ou partie:
* des enchaînements de nucléotides suivants: 
5'-CTGTGGCCGATCAGGATGGTTTTGTCGTGC-3'
..A.......................A...
T.............C...............
.....A..A.....C...............
.................A............
 ou leurs séquences complémentaires,
* ou d'une séquence dérivée de la séquence D, notamment par suppression et/ou substitution et/ou addition de nucléotides, et susceptible de s'hybrider avec la séquence complémentaire du fragment délimité par les positions 672 et 701 de la séquence représentée sur la figure 2, ou la séquence complémentaire de cette séquence dérivée.

A titre illustratif, on entend en outre par séquence dérivée dans ce qui précède, et ce qui suit, toute séquence comprenant des nucléotides tels que l'uracile, l'inosine, le 7 deaza 2' desoxyguanosine triphosphate (cdGTP) etc..., et correspondant donc soit à un ADN soit à un ARN modifié.

Les séquences nucléotidiques susmentionnées peuvent être avantageusement marquées, notamment de manière radioactive, enzymatique, par un composé fluorescent, par liaison à une molécule antigènique (susceptible d'être reconnue par des anticorps) ou à toute autre molécule susceptible d'être directement ou indirectement détectée à l'aide de réactifs, cette liaison pouvant éventuellement être effectuée par l'intermédiaire d'un bras espaceur, notamment par l'intermédiaire d'une séquence nucléotidique constituée d'environ 5 à 100 nucléotides située à l'extrémité 3' ou 5' de ces séquences.

A titre d'exemples de molécules directement ou indirectement détectables liées aux séquences nucléotidiques susmentionnées, on peut citer l'acétylamblofluorène, la biotine (détectée à l'aide de l'avidine, la streptavidine) ou encore la digoxigènine.

Les séquences nucléotidiques susmentionnées peuvent être utilisées par paires pour former ce que l'on appellera dans la suite de ce texte des couples d'amorces, ces amorces permettant l'amplification (ou encore la multiplication) du nombre de copies de tout ou partie des gènes, ou des copies des ARN correspondants et de leurs dérivés nucléotidiques par transcription inverse, codant des PL chez les erwinias pectinolytiques, notamment selon la technique PCR qui sera détaillée plus loin ou toute autre technique faisant appel à l'hybridation entre séquences nucléotidiques et permettant de détecter un nombre initialement faible de séquences nucléotidiques déterminées (ou de leurs séquences complémentaires), par multiplication du nombre de copies de ceux-ci ou de leurs dérivés, ces amorces étant le cas échéant marquées, notamment de la manière indiquée ci-dessus.

Avantageusement, les amorces selon l'invention sont constituées d'environ 5 à environ 100 nucléotides, et de préférence d'environ 15 à environ 30 nucléotides, et sont susceptibles de s'hybrider avec une région de ces gènes codant des PL chez ces erwinias pectinolytiques, cette région étant différente selon les amorces au sein d'un même couple, de manière à permettre l'amplification soit des gènes entiers, soit de fragments de ces gènes, ces fragments étant constitués d'environ 150 à environ 700 nucléotides, et avantageusement d'environ 400 à environ 500 nucléotides.

L'invention a plus particulièrement pour objet les couples d'amorces pour l'amplification génique spécifique des *Erwinia carotovora (Ec),* caractérisés en ce que l'une des amorces de ces couples est choisie parmi les séquences comprenant tout ou partie de la séquence nucléotidique A, ou une séquence dérivée, telle que définie ci-dessus, tandis que l'autre amorce est choisie parmi les séquences comprenant tout ou partie de la séquence nucléotidique B, ou une séquence dérivée, telle que définie ci-dessus.

L'invention a également plus particulièrement pour objet les couples d'amorces pour l'amplification génique spécifique des *Ech,* caractérisés en ce que l'une des amorces de ces couples est choisie parmi les séquences comprenant tout ou partie de la séquence nucléotidique C, ou une séquence dérivée, telle que définie ci-dessus, tandis que l'autre amorce est choisie parmi les séquences comprenant tout ou partie de la séquence nucléotidique D, ou une séquence dérivée, telle que définie ci-dessus.

De préférence les amorces selon l'invention comprennent les 3, et de préférence les 5 derniers nucléotides des extrémités 3' des séquences (A, B, C et D) décrites ci-dessus.

Les séquences nucléotidiques susmentionnées peuvent également être utilisées en tant que sondes, le cas échéant marquées, notamment de la manière indiquée ci-dessus, ces sondes étant susceptibles de s'hybrider avec tout ou partie des gènes, ou des ARN correspondants, ou de leurs dérivés nucléotidiques par transcription inverse, codant des pectate-lyases chez les erwinias pectinolytiques.

Avantageusement les sondes de l'invention sont constituées d'environ 10 à environ 500 nucléotides.

Dans le cadre de l'utilisation des séquences susmentionnées en tant que sondes pour l'identification et la détection des *Erwinia carotovora,* des séquences particulièrement préférées comprennent au moins environ 10 nucléotides de la séquence délimitée par les nucléotides situés aux positions 1613 et 1707 de la séquence représentée sur la figure 1, ou au moins environ 10 nucléotides ayant un minimum de 40% d'homologie avec la séquence susmentionnée, ou encore toute séquence susceptible de rester hybridée avec celles définies ci-dessus, notamment dans les conditions d'hybridation décrites plus haut.

A titre illustratif, les séquences A et B, ou toute séquence dérivée, telle que décrite ci-dessus, représentent des sondes particulièrement avantageuses pour la détection d'*Erwinia carotovora,* et plus particulièrement d'*Ecc,*d'*Eca,* d'*Eco* et d'*Ecw,* ou de toute espèce, sous-espèce ou pathovar, actuels ou dérivés ultérieurement par modification de la taxonomie actuelle.

Dans le cadre de l'utilisation des séquences susmentionnées en tant que sondes pour l'identification et la détection des *Erwinia chrysanthemi,* des séquences particulièrement préférées sont constituées d'au moins environ 10 nucléotides:
- de la séquence représentée sur la figure 2 et délimitée par les nucléotides situés aux positions 295 et 701 du *gène pelE* de la souche EC16 d'*Ech*,
- ou de la séquence délimitée par les nucléotides situés aux positions 130 à 970 du gène *pelA* de la souche 3937 d'*Ech* décrite dans l'article de Favey *et al.* susmentionné,
ou d'au moins 10 nucléotides ayant un minimum de 40% d'homologie avec les séquences susmentionnées, ou encore toute séquence susceptible de rester hybridée avec celles définies ci-dessus, notamment dans les conditions d'hybridation décrites plus haut.

A titre illustratif, les séquences C et D, ou toute séquence dérivée, telles que décrites ci-dessus, représentent des sondes particulièrement avantageuses pour la détection d'*Ech* ou de toute espèce, sous-espèce ou pathovar, actuels ou dérivés ultérieurement par modification de la taxonomie actuelle.

Le procédé d'identification et de détection des erwinias pectinolytiques selon l'invention, comprend avantageusement les étapes suivantes:
- le traitement d'un échantillon prélevé dans l'eau, ou le sol, ou chez un hôte, notamment chez les plantes et les semences, de manière à rendre le génome de ces erwinias accessible aux sondes et/ou aux amorces de l'invention, et le cas échéant à toute ADN ou ARN polymérase permettant de répliquer les deux brins de l'ADN génomique, ou l'ARN en dérivant, ce traitement étant notamment effectué par ébullition, macération (dans un liquide tel que l'eau), broyage, sonication, notamment en présence de détergent (par exemple laurylsulfate, Sodium Dodecyl Sulfate, Triton X100, (marque déposée)), d'antioxydant, de chélateurs, d'alcali, et/ou de composés éliminant des inhibiteurs tels que les polyphénols (polyvinylpyrrolidone par exemple), ou les polysaccharides (bromure de cétyltriméthylammonium par exemple),
- l'amplification du nombre de copies de gènes codant des PL, ou de fragments de ces gènes, susceptibles d'être présents dans cet échantillon, à l'aide de couples d'amorces (ou de leurs séquences dérivées ou complémentaires) tels que définis ci-dessus,
- la détection de fragments amplifiés lors de l'étape précédente, témoignant de la présence de gènes codant des PL, et donc de la présence d'erwinias pectinolytiques dans l'échantillon étudié.

L'étape d'amplification génique peut être réalisée in vitro ou in vivo par toute méthode utilisant les techniques classiques d'amplification enzymatique de l'ADN ou de l'ARN, telles que la technique LCR (Ligase Chain Reaction) décrite notamment dans la revue P.N.A.S., USA, 88, pp. 189-193 (1991), ou la technique "Qβ Replicase" décrite dans la revue Biotechnology (Vol.6, octobre 1988), ou avantageusement la technique PCR telle que décrite notamment dans les demandes de.brevet européen de Cetus (n° 200 362,201 184, 229 701 et 258 017), ou encore la technique 3SR décrite par Fahy et *al.* dans *PCR Meth. Appl.* 1, 25-33 (1991), ou les techniques dérivées de ces dernières et toute méthode visant à amplifier in vitro les séquences nucléotidiques.

A titre illustratif, l'amplification de ces gènes, ou fragments de gènes, comprend avantageusement les étapes suivantes:
- la prédénaturation de l'ADN double brin en ADN mono-brin, de préférence dans un tampon constitué de Tris-HCl 10 mM pH 8,3, 50 mM KCl, 1,5 mM MgCl₂, 0,01% de gélatine, de co-facteurs de l'ADN-polymérase, notamment d'ions Mg²⁺ et K⁺, des 4 désoxynucléotides constitutifs des ADN (dCTP, dATP, dGTP, dTTP), et des couples d'amorces tels que définis ci-dessus, par chauffage entre environ 80°C et environ 110°C, avantageusement à 100°C; cette étape peut-être réalisée pendant environ 2 mn à environ 7 mn, avantageusement pendant 5 mn.
- l'amplification proprement dite par addition au milieu obtenu à l'étape précédente d'ADN polymérase thermorésistante par exemple, et
   * chauffage à environ 94°C, ce qui correspond à l'étape de dénaturation proprement dite; cette étape peut-être réalisée pendant environ 10 s à environ 2 mn,
   * puis chauffage entre environ 60°C et environ 76°C, ce qui correspond à l'étape d'hybridation des couples d'amorces avec les gènes codant des PL, ou des fragments de ces gènes, susceptibles d'être présents dans l'échantillon biologique étudié; cette étape peut-être réalisée pendant environ 10 s à environ 3 mn,
   * et enfin chauffage entre environ 50°C et environ 76°C, ce qui correspond à l'étape d'élongation des amorces, hybridées à l'étape précédente, l'une vers l'autre, produisant ainsi des séquences nucléotidiques complémentaires de tout ou partie des séquences génomiques des *Erwinias* pectinolytiques, ces dernières séquences étant délimitées par les nucléotides s'hybridant avec les amorces susmentionnées; cette étape peut-être réalisée pendant environ 10 s à environ 3 mn,
- la répétition de l'étape d'amplification précédente entre environ 20 et environ 50 fois, avantageusement entre 25 et 35 fois,
- le cas échéant, la récupération d'une partie aliquote du milieu obtenu à la fin de l'étape d'amplification précédente, afin de réaliser une nouvelle amplification selon la méthode décrite ci-dessus.

Il va de soi que tous les intervalles de temps précisés dans les méthodes détaillées ci-dessus, peuvent varier en fonction de l'appareillage (dit cycleur ou thermocycleur) utilisé.

Bien entendu, dans le cadre de l'amplification du nombre de copies par multiplication d'un fragment d'ARN, les 4 désoxynucléotides utilisés dans la méthode précédente sont dUTP, dCTP, dGTP, dATP.

Selon un autre mode de réalisation de l'invention, la sensibilité de la détection peut être améliorée en poursuivant le procédé décrit ci-dessus par une amplification du type "nested PCR", décrite dans "PCR A Practical Approach" Edited by M.J.McPherson, P.Quirke, G.R.Taylor, page 42, (1991) Oxford University Press, en utilisant des amorces internes au fragment précédemment amplifié, ce qui conduit à l'amplification du nombre de copies d'un fragment d'ADN ou d'ARN plus court que celui dont le nombre de copies a été initialement amplifié.

La détection des gènes ou fragments de gènes susmentionnés peut être réalisée par électrophorèse sur gel de tout ou partie du milieu réactionnel dans lequel l'amplification a été effectuée. La visualisation d'un amas important (plus ou moins individualisé) de séquences en un point spécifique du gel, et correspondant au nombre de copies de tout ou partie des gènes ainsi amplifiées, est corrélable à la présence des gènes susmentionnés dans l'échantillon étudié.

Avantageusement, le nombre amplifié de copies de tout ou partie des gènes sus-mentionnés, est susceptible d'être détecté à l'aide de sondes telles que décrites ci-dessus, notamment selon le procédé décrit ci-dessous.

Dans le cas d'utilisation de sondes, la détection des fragments de gènes amplifiés peut être réalisée soit à l'aide d'une sonde (hybridation simple), notamment par utilisation d'amorces ou nucléotides marqués, soit à l'aide de deux sondes (hybridation double).

A titre illustratif, le procédé de l'invention par hybridation double, est avantageusement réalisé de la manière suivante:
- après avoir rendu accessible le génome des bactéries dans les échantillons, on procède à l'amplification selon la technique décrite ci-dessus, puis on procède :
- à la fixation sur un support solide (tel qu'une membrane ou des puits de plaques de microtitration comme celles couramment utilisées dans la technique dite ELISA, et adaptées ou non aux acides nucléiques) d'une séquence nucléotidique "piège", choisie parmi les sondes de l'invention décrites ci-dessus, avec laquelle les susdits gènes, ou fragments de ces gènes, sont susceptibles de s'hybrider, notamment dans les conditions définies ci-dessus, cette fixation étant réalisée selon des techniques usuelles décrites notamment par Maniatis *et al.,* 1982,
- au rinçage du support solide,
- à la saturation des sites réactifs libres du support,
- à l'incubation des séquences pièges, ainsi fixées, avec l'ADN (ou ARN) génomique amplifié, provenant de l'échantillon biologique préalablement traité de la manière indiquée ci-dessus, dans des conditions permettant l'hybridation de l'ADN (ou ARN) susmentionnés avec lesdites séquences pièges, notamment dans les conditions d'hybridation définies plus haut,
- au rinçage du support solide, notamment à l'aide de 6xSSC à 65°C (ou 3xSSC à 65°C, ou 0,1xSSC si nécessaire, à 68°C) pendant 15 mn à deux reprises,
- à l'incubation des séquences d'ADN ou ARN susmentionnées, hybridées à l'étape précédente avec les séquences pièges, avec une ou plusieurs sondes selon l'invention, ces sondes étant choisies de manière à ce qu'elles ne soient pas complémentaires des séquences pièges susmentionnées, dans des conditions permettant l'hybridation des séquences d'ADN ou ARN amplifiées susmentionnées avec lesdites sondes, notamment dans les conditions d'hybridation définies plus haut,
- au rinçage du support solide, notamment dans les conditions susmentionnées,
- à la détection des éventuelles sondes étant restées fixées, par hybridation avec les séquences nucléotidiques amplifiées, sur le support solide après l'étape précédente, témoignant alors de la présence d'ADN (ou ARN) génomique susmentionnés dans l'échantillon biologique, cette détection étant réalisée soit directement lorsque les susdites sondes sont marquées de manière radioactive ou fluorescente, soit indirectement par l'intermédiaire de réactifs eux-mêmes susceptibles de reconnaître ces sondes, notamment à l'aide d'anticorps ou autres réactifs décrits précédemment, dans le cas où ces sondes sont marquées par des antigènes ou des molécules susceptibles d'être respectivement reconnus par ces anticorps ou ces réactifs.

Avantageusement, les deux sondes utilisées dans le cadre de la détection par hybridation double peuvent être à spécificité variable. Par exemple, on peut utiliser à titre de sonde piège, une sonde spécifique d'une espèce déterminée, et plus particulièrement de l'espèce *Erwinia carotovora* ou de l'espèce *Erwinia chrysanthemi.* La seconde sonde utilisée dans le procédé par hybridation double décrit ci-dessus, pourra être choisie parmi les sondes spécifiques des sous-espèces ou pathovars des espèces susmentionnées.

D'autres méthodes de détection ou de dosage, en phase solide ou liquide, peuvent bien entendu être envisagées dans le cadre de la présente invention.

Ces méthodes sont décrites notamment dans l'article de Bloch, 1991, qui traite de la technique PCR en général et d'une méthode de dosage par co-amplification applicable au dosage des erwinias pectinolytiques présentes dans l'échantillon étudié, ou dans l'article de Barany, 1991, traitant de la LCR et de la PCR, ou encore dans l'article de Gilliland et al., 1990, traitant d'une méthode PCR par compétition pour la quantification d'ARNm également applicable dans le cadre de la présente invention.

L'invention vise plus particulièrement un procédé tel que décrit ci-dessus, permettant l'identification et la détection d'*Erwinia carotovora,* et plus particulièrement d'*Ecc,* d'*Eca,* d'*Eco*et d'*Ecw,* ou de toute espèce, sous-espèce ou pathovar, actuels ou dérivés ultérieurement par modification de la taxonomie actuelle, ce procédé étant caractérisé en ce que:
- les couples d'amorces utilisés sont constitués d'une séquence nucléotidique A ou dérivée, et d'une séquence nucléotidique B ou dérivée, telles que définies ci-dessus,
- les séquences pièges et les sondes sont choisies parmi les séquences comprenant au moins 10 nucléotides de la séquence délimitée par les nucléotides situés aux positions 1231 et 1665 de la séquence représentée sur la figure 1.

Avantageusement, les étapes de dénaturation, d'hybridation et d'élongation du procédé susmentionné, sont respectivement réalisées à 94°C pendant 1 mn, à 65°C pendant 1 mn, et à 72°C pendant 1 mn 30 s.

L'invention concerne également un procédé tel que décrit ci-dessus, permettant l'identification et la détection d'*Ech* ou de toute espèce, sous-espèce ou pathovars, actuels ou dérivés ultérieurement par modification de la taxonomie actuelle, ce procédé étant caractérisé en ce que :
- les couples d'amorces utilisés sont constitués d'une séquence nucléotidique C ou dérivée, et d'une séquence nucléotidique D ou dérivée, telles que définies ci-dessus,
- les séquences pièges et les sondes sont choisies parmi les séquences comprenant au moins 10 nucléotides de la séquence délimitée par les nucléotides situés aux positions 295 et 701 de la séquence représentée sur la figure 2.

Avantageusement, dans le cas du procédé susmentionné, l'étape de dénaturation est réalisée à 94°C pendant 1 mn, et les étapes d'hybridation et d'élongation sont regroupées en une étape réalisée à 72°C pendant 2 mn.

L'invention a également pour objet l'application des procédés d'identification et de détection susmentionnés à la mise en oeuvre d'une méthode de diagnostic de l'éventuelle apparition (encore désignée par méthode de diagnostic précoce) de pathologies causées par les erwinias pectinolytiques, et plus particulièrement par les *Erwinia carotovora* et les *Erwinia chrysanthemi,* chez un hôte tel que défini ci-dessus, cet hôte étant susceptible d'être un porteur apparemment sain de telles bactéries et se trouvant en phase latente d'infection.

Le résultat de la méthode de diagnostic susmentionnée permet d'évaluer le risque que présente un hôte susceptible d'être porteur d'erwinias pectinolytiques, et plus particulièrement d'*Ec* et d'*Ech,* de développer et le cas échéant, de transmettre, ou non, une pathologie causée par ces bactéries.

L'invention vise également l'application des procédés décrits ci-dessus au dépistage des erwinias pectinolytiques dans tout milieu susceptible de les contenir, notamment dans le sol et l'eau, et de représenter ainsi des sources possibles de contamination.

Par erwinias pectinolytiques détectées dans le cadre des méthodes de diagnostic et de détection susmentionnées, il faut entendre à la fois les souches naturelles de ces bactéries, ainsi que les souches bactériennes, et autres organismes, modifiés, notamment par voie du génie génétique.

Les pathologies en phase latente d'infection susceptibles d'être dépistées dans le cadre de la présente invention sont plus particulièrement:
- des maladies génériques dites de pourritures (humides ou molles), à développement localisé et/ou systémique, de plantes entières ou d'organes au champ ou en conservation ou encore en survie. A titre d'exemples, on peut citer les pourritures de la pomme de terre (plantes ou tubercules), de chicons d'endives ou d'endives en forçage ou stockées, de Saintpaulia, de toute salade, d'organes végétaux ayant éventuellement subi (encore désignés par produits de 4° gamme) des modifications d'eux-mêmes ou de leur environnement (atmosphères modifiées, lavages, désinfection etc...),
- de pourritures dites systémiques individualisées telles que la jambe noire ou le manque à la levée de la pomme de terre,
- de flétrissement se caractérisant par un port inhabituel des plantes, généralement observé après obstruction des vaisseaux du végétal, notamment par empêchement de la circulation d'une ou des deux sèves (notamment chez la pomme de terre et l'oeillet).

L'invention a également pour objet des kits pour la mise en oeuvre d'un procédé ou d'une méthode de diagnostic selon l'invention, ces kits comprenant au moins une séquence nucléotidique choisie parmi celles définies ci-dessus et correspondant à tout ou partie de gènes qui, au sein des génomes des erwinias pectinolytiques, codent des pectate-lyases.

L'invention a plus particulièrement pour objet des kits comprenant:
- au moins un couple d'amorces dont l'une est constituée de tout ou partie de la séquence A ou d'une séquence dérivée de cette dernière, et l'autre est constituée de tout ou partie de la séquence B ou d'une séquence dérivée de cette dernière, telles que décrites ci-dessus, ou de leurs séquences complémentaires, ce couple d'amorces permettant l'amplification génique de séquences nucléotidiques d'*Erwinia carotovora,* notamment des *Eca, Eco, Ecc* et *Ecw,*
- une (ou plusieurs) sonde(s) susceptibles de s'hybrider avec tout ou partie des fragments de gènes amplifiés à l'aide des couples d'amorces susmentionnés, et plus particulièrement des sondes comprenant au moins 10 nucléotides de la séquence délimitée par les nucléotides situés aux positions 1231 et 1665 de la séquence représentée sur la figure 1

L'invention vise également des kits comprenant:
- un couple d'amorces dont l'une est constituée de tout ou partie d'une séquence C ou d'une séquence dérivée de cette dernière, et l'autre est constituée de tout ou partie d'une séquence D ou d'une séquence dérivée de cette dernière, telles que décrites ci-dessus, ou de leurs séquences complémentaires, ce couple d'amorces permettant l'amplification génique de séquences nucléotidiques d'*Erwinia chrysanthemi,*
- une (ou plusieurs) sonde(s) susceptibles de s'hybrider avec tout ou partie des fragments de gènes amplifiés à l'aide des couples d'amorces susmentionnés, et plus particulièrement des sondes comprenant au moins 10 nucléotides de la séquence délimitée par les nucléotides situés aux positions 295 et 701 de la séquence représentée sur la figure 2.

Les kits selon l'invention peuvent également comprendre:
- une ADN ou ARN polymérase thermorésistante,
- un milieu réactionnel avantageusement constitué de Tris-HCl 10 mM pH 8,3, 50 mM KCl, 1,5 mM MgCl₂, 0,01% de gélatine, de co-facteurs de l'ADN-polymérase, notamment d'ions Mg²⁺ et K⁺, et des 4 désoxynucléotides constitutifs des ADN (dCTP, dATP, dGTP, dTTP) ou des ARN (dCTP, dATP, dGTP, dUTP).

L'invention concerne également l'utilisation de séquences nucléotidiques correspondant à tout ou partie de gènes qui, au sein des génomes des bactéries pectinolytiques du genre *Erwinia,* et plus particulièrement d'*Erwinia carotovora* et d'*Erwinia chrysanthemi,* codent des pectate-lyases, pour la mise en oeuvre d'un procédé d'identification des sous-espèces, et/ou des souches ou pathovars des espèces ou sous-espèces, des différentes bactéries du genre *Erwinia.*

L'invention a également pour objet l'utilisation de séquences nucléotidiques, telles que définies ci-dessus, correspondant à tout ou partie de gènes qui, au sein des génomes des bactéries pectinolytiques du genre *Erwinia,* codent des pectate-lyases, pour la mise en oeuvre d'une méthode de détection du polymorphisme des fragments amplifiés, celui-ci pouvant par exemple être mis en évidence par le polymorphisme de taille des fragments de restriction issus des gènes susmentionnés amplifiés, ces fragments (encore désignés fragments de référence dans ce qui suit) étant caractéristiques de chacune des espèces, sous-espèces ou pathovars d'une même espèce de bactéries pectinolytiques du genre *Erwinia.*

Avantageusement, la détection des fragments amplifiés sera effectuée par hybridation avec des sondes à spécificité plus ou moins large en fonction des espèces, sous-espèces ou pathovars des erwinias pectinolytiques. Cette détection peut être réalisée selon la méthode RFLP (Restriction Fragment Length Polymorphism), ou par détection en phase solide (par hybridation simple ou double telles que décrites ci-dessus) telle que par exemple celle décrite par Erlich, Gibbs et Kazajian, Eds (1989) dans Current Communications in Molecular Biology. Polymerase Chain Reaction. Cold Spring Harbor laboratory Press NY.

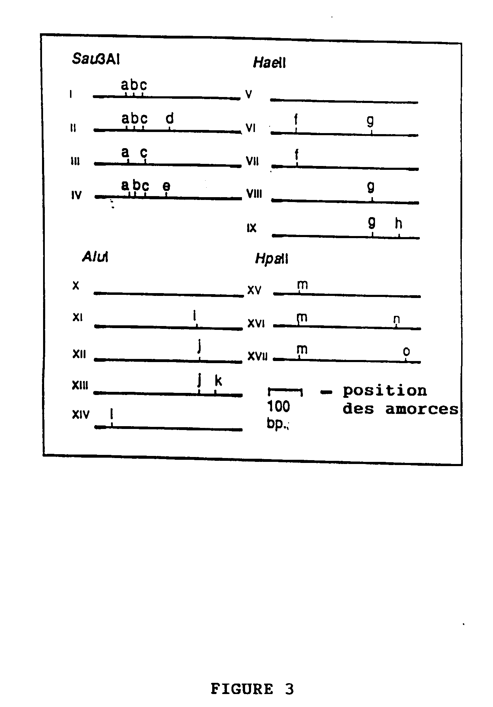

A ce titre, l'invention concerne plus particulièrement les fragments de référence obtenus, chez *Erwinia carotovora,* par restriction avec les enzymes Sau3A1 et *Hae*II et représentés sur la figure 3.

L'invention vise plus particulièrement les sondes aptes à mettre en évidence le polymorphisme observé avec *Sau3*A1 et *Hae*II, dans le cas d'*Eca.*

L'invention a plus particulièrement pour objet un procédé d'identification et de détection d'une souche d'une espèce ou sous-espèce ou pathovar déterminé de bactéries pectinolytiques du genre *Erwinia* chez un hôte, notamment chez les plantes et les semences, ce procédé étant réalisé par la mise en oeuvre d'un des procédés d'identification et de détection décrits précédemment et comprenant une étape de détection de la souche susmentionnée.

Cette étape d'identification des souches des espèces, sous-espèces et pathovars susmentionnés, peut représenter une étape supplémentaire dans le cadre des procédés d'identification et de détection susmentionnés. Les procédés sus-mentionnés se font alors en deux temps; détection de la présence éventuelle d'erwinias pectinolytiques, suivie de la détection ou de la discrimination de l'espèce, sous-espèce ou pathovar en question.

Cette étape de détection de ces souches peut également remplacer l'étape de détection des erwinias dans les procédés susmentionnés, de manière à ce que l'espèce ou sous-espèce en question éventuellement présente dans le milieu biologique soit directement détectée.

La détection des souches des espèces, sous-espèces ou pathovars, est avantageusement réalisée de la manière suivante:
- mise en présence des gènes ou fragments de gènes codant des PL chez les erwinias pectinolytiques susceptibles d'être présentes dans l'échantillon étudié, et dont le nombre de copies a été le cas échéant amplifié, avec une (ou plusieurs) enzyme(s) de restriction susceptible(s) de couper ces gènes ou fragments de gènes en un (ou plusieurs) site(s) spécifique(s) d'une espèce, sous-espèce ou pathovar déterminé d'*Erwinia,* puis comparaison de la taille des fragments de restriction obtenus à l'étape précédente avec celle de fragments de référence caractéristiques de l'espèce ou sous-espèce déterminée susmentionnée, tels que les fragments de référence décrits ci-dessus, notamment par électrophorèse sur gel,
- ou détection de sites de restriction polymorphes à l'aide de sondes spécifiques des souches des sous-espèces ou pathovars en question, notamment selon la méthode décrite à la page 200 du livre d'Erlich "PCR Technology" (1989) Stockton Press,
- ou encore par comparaison de la température de fusion de domaines des fragments obtenus avec celle des fragments de référence; cette technique peut avantageusement être réalisée par électrophorèse sur gel, en milieu dénaturant, ou non, homogène ou en gradient continu ou discontinu (composé par exemple d'urée, de formamide etc ...), ou en gradient de température, notamment selon les techniques décrites dans Current Communication in Molecular Biology, Polymerase Chain Reaction, édité par H. A. Erlich, R. Gibbs et H. H. Kazazian, Cold Spring Harbor Laboratory Press, 1989.

La détection peut également être réalisée à l'aide d'amorces prises dans les régions polymorphes mises en évidence par les enzymes de restriction susmentionnées.

Avantageusement la détection des souches d'espèces, des sous-espèces ou des pathovars des bactéries pectinolytiques du genre *Erwinia* est réalisée selon la technique LCR telle que décrite ci-dessus, ou une technique dérivée de (ou apparentée à) cette dernière, notamment de la manière suivante:
- mise en présence des gènes ou fragments de gènes codant des PL, dont le nombre de copies a été le cas échéant amplifié, lors de la mise en oeuvre d'une méthode de diagnostic de l'invention telle que décrite ci-dessus, avec un (ou plusieurs) couple(s) de séquences oligonucléotidiques, chacune de ces séquences étant constituée d'environ 10 à environ 30 nucléotides et étant susceptible de s'hybrider de part et d'autre d'un (ou plusieurs) nucléotide(s) de ces gènes ou fragments de gènes, ce (ou ces) dernier(s) nucléotide(s) étant caractéristique(s) de la souche de l'espèce, sous-espèce ou pathovar d'*Erwinia* en question, et en particulier de part et d'autre des séquences caractéristiques des sites de restriction des enzymes utilisées pour la détermination de cette souche (espèce, sous-espèce ou pathovar), dans des conditions permettant l'hybridation de ces gènes ou fragments de gènes avec ce (ou ces) couple(s) de séquences, notamment dans les conditions d'hybridation définies ci-dessus,
- addition d'une ligase, par exemple thermorésistante, de sorte que ce (ou ces) couple(s) de séquences puisse(nt) donner naissance à une ou des séquences uniques constituées de chacune des deux séquences de ces couples et du (ou des) nucléotide(s) complémentaire(s) de celui (ou ceux) susmentionné(s) caractéristique(s) de la souche d'*Erwinia* en question, sous l'action de cette ligase, lorsque les deux séquences du (ou des) couple(s) sont hybridées aux gènes ou fragments de gènes susmentionnés,
- amplification par ligation, avec les étapes nécessaires de dénaturation, d'hybridation et d'aboutage, du nombre de copies de ces séquences uniques, notamment suivant les techniques décrites ci-dessus,
- détection de ces séquences uniques, cette détection étant avantageusement réalisée par électrophorèse, ou de préférence à l'aide de sondes spécifiques marquées ou non (hybridation simple) ou susceptibles d'être reconnues par des réactifs marqués (hybridation double par exemple) de la manière décrite ci-dessus.

Par nucléotides caractéristiques d'une espèce ou sous-espèce d'*Erwinia,* on entend tout nucléotide qui, dans une position déterminée d'un gène codant une pectate-lyase de cette espèce, sous-espèce ou pathovar d'*Erwinia,* est différent des nucléotides situés à la même position dans ce même gène chez les autres espèces, sous-espèces ou pathovars d'*Erwinia.* Ces nucléotides présentent également la propriété de modifier la capacité d'une enzyme de restriction à couper le fragment cible ou amplifié de l'échantillon étudié.

L'invention concerne plus particulièrement les couples de séquences nucléotidiques tels que décrits ci-dessus pour la mise en oeuvre de la méthode susmentionnée de détection des espèces, sous-espèces ou pathovars de bactéries du genre *Erwinia,* ces séquences étant avantageusement choisies parmi celles bordant les sites *Sau*3A1 et *Hae*II des gènes codant des PL chez ces espèces, sous-espèces ou pathovars d'*Erwinia.*

L'invention a également pour objet des kits pour la mise en oeuvre des méthodes de diagnostic susmentionnées de pathologies causées spécifiquement par une espèce, sous-espèce ou pathovar déterminés d'*Erwinia,* ces kits comprenant, outre tout ou partie des kits décrits ci-dessus:
- une (ou plusieurs) sonde(s) oligonucléotidique(s), marquée(s) ou non, fixée(s) ou non sur un support solide, spécifique(s) d'une région nucléotidique caractéristique d'une espèce, sous-espèce ou pathovar déterminé de bactéries du genre *Erwinia,*
- le cas échéant, une ou plusieurs enzymes de restriction, et avantageusement des fragments de restriction de référence tels que décrits ci-dessus,
- et/ou une ligase et un (ou plusieurs) couple(s) de séquences oligonucléotidiques, tel(s) que décrit(s) ci-dessus, susceptibles de s'hybrider de part et d'autre d'un (ou plusieurs) nucléotide(s) situé(s) dans des gènes ou fragment de gènes codant des pectate-lyases, et étant caractéristique(s) d'une espèce, sous-espèce ou pathovar d'*Erwinia.*

L'invention concerne également l'utilisation des séquences nucléotidiques définies ci-dessus pour la mise en oeuvre d'un procédé de détection, le cas échéant dans un but de diagnostic, chez un hôte ou dans un milieu tels que définis ci-dessus, de tout micro-organisme ou toute cellule, d'eucaryotes par exemple, dans les génomes desquels les séquences nucléotidiques décrites ci-dessus, ont été introduites de façon transitoire ou définitive. A nouveau, un tel procédé est avantageusement réalisé suivant l'un des procédés d'identification et de détection décrits ci-dessus.

A ce titre, l'invention a également pour objet l'utilisation des séquences nucléotidiques définies ci-dessus correspondant à tout ou partie des gènes codant des PL chez les erwinias pectinolytiques, en tant que marqueurs de cellules, d'eucaryotes par exemple, ou de micro-organismes génétiquement modifiés par introduction dans leur génome de ces séquences nucléotidiques, ou d'hôtes cellulaires infectés (ou transformés) par de tels micro-organismes, notamment pour le suivi de l'évolution de ces cellules, micro-organismes et hôtes cellulaires dans un environnement déterminé.

L'invention concerne également tout procédé d'obtention des séquences nucléotidiques susmentionnées.

Concernant la préparation des séquences nucléotidiques de l'invention, celle-ci peut être effectuée soit par un procédé chimique, soit par d'autres procédés, comme l'amplification par LCR ou PCR ou autre méthode d'amplification génique.

Un mode de préparation approprié des séquences nucléotidiques (comportant au maximum environ 200 nucléotides, ou paires de bases (pb) lorsqu'il s'agit de séquences nucléotidiques bicaténaires) de l'invention par voie chimique comprend les étapes suivantes:
- la synthèse d'ADN en utilisant par exemple la méthode automatisée β-cyanéthyl phosphoramidite décrite dans Bioorganic Chemistry 4; 274-325, 1986,
- le clonage des ADN ainsi obtenus dans un vecteur, notamment plasmidique, approprié et la récupération des ADN par hybridation avec une sonde appropriée, ou par électrophorèse, généralement après restriction, suivie d'une élution des fragments.

Un mode de préparation, par voie chimique, de séquences nucléotidiques de longueur supérieure à environ 200 nucléotides - ou pb (lorsqu'il s'agit de séquences nucléotidiques bicaténaires) comprend les étapes suivantes:
- l'assemblage, par exemple par aboutage (ou ligation, par action d'une ligase), d'oligonucléotides synthétisés chimiquement, pourvus à leurs extrémités de sites de restriction adéquats, selon le principe décrit dans Proc. Nat. Acad. Sci. USA, 80; 7461-7465, 1983,
- le clonage des ADN ainsi obtenus dans un vecteur, notamment plasmidique, approprié et la récupération des ADN par hybridation avec une sonde appropriée, ou par électrophorèse, généralement après restriction, suivie d'une élution des fragments.

La préparation des séquences nucléotidiques de l'invention peut également être réalisée par voie biologique, notamment après transformation et clonage d'hôtes cellulaires susceptibles de contenir ces séquences nucléotidiques, et de permettre la multiplication de ces dernières.

Un autre procédé d'obtention consiste en l'amplification du nombre de copies d'une séquence nucléotidique déterminée à l'aide d'amorces appropriées telles que décrites ci-dessus, notamment selon la technique PCR réalisée à partir de gènes ou fragments de gènes codant des PL chez les erwinias pectinolytiques.

L'invention sera davantage détaillée dans les exemples qui suivent de mise en oeuvre de méthodes de détection par PCR de bactéries pectinolytiques du genre *Erwinia.*

### A) Matériel et méthodes

Souches bactériennes: des souches de sous espèces *d'Erwinia carotovora,* et de l'espèce *Erwinia chrysanthemi,* ainsi que d'autres micro-organismes ont été utilisés (voir Tableaux 1 et 2).

### Isolement d'ADN Chromosomique:

Les bactéries sont cultivées à 30°C dans un milieu de Luria (milieu L) (Miller, 1972) pendant une nuit. Deux millilitres de cultures sont centrifugés pour l'extraction de l'ADN par la méthode de Klotz et Zimm (1972) modifiée : les réactifs sont réduits d'un facteur 10.

Analyse de séquences: des résultats publiés ont été utilisés: *pelY* (Manulis *et al.,* 1988), *pelB* (Lei *et al.,* 1987), *pelA* (Lei et *al.,* 1988), *polI* (Ito *et al.,* 1988), *pel153* (Trollinger *et al.,* 1989), *pelA, pelC, pelE* et *pelD* (Tamaki *et al.,* 1988), *pelA, pelD* et *pelE* (Van Gijsegem, 1989), *pelB* (Schoedel et Collmer 1986), *pelA* (Favey *et al.,* 1992) *et pelB* (Hinton *et al.,* 1989).

Les séquences nucléotidiques des gènes *pel* ont été étudiées dans le cadre de lecture ouverte (ORF), alignées par paires, en utilisant un ensemble de programmes d'analyse de séquences (GCG: Genetic Computer Group, Université du Wisconsin). Les régions conservées dans différentes souches de sous espèces *d'Erwinia carotovora (Ec),* et de l'espèce *Erwinia chrysanthemi,* et celles différentes des autres espèces ont été plus particulièrement étudiées.

Les amorces ont été choisies à l'aide des critères suivants:
- ces amorces doivent être suffisemment éloignées pour permettre l'amplification de fragments porteurs de polymorphisme,
- ces amorces ne doivent cependant pas être trop éloignées de manière à assurer une amplification rapide des fragments en question,
- les 3 (et si possible 5) derniers nucléotides de l'extrémité 3' des amorces doivent être invariants et donc strictement complémentaires de la partie correspondante des fragments à amplifier.

Les amorces trouvées dans le cadre de la présente invention sont distantes d'environ 400 à 500 nucléotides des fragments à amplifier.

Parmi les amorces trouvées, les amorces A, B, C et D susmentionnées représentent des amorces particulièrement avantageuses.

Dans le cas d'*Ec,* deux oligonucléotides (24 mères) distants d'environ 400 pb sont choisis comme amorces de PCR:

5'-TTACCGGACGCCGAGCTGTGGCGT-3' (amorce A) et
5'-CAGGAAGATGTCGTTATCGCGAGT-3' (amorce B).

Dans le cas d'*Ech,* deux séries d'oligonucléotides (l'une de 23 mères, l'autre de 30 mères) distants d'environ 400 pb sont choisis comme amorces de PCR:

5'-GATCAGAAAGCCCGCAGCCAGAT-3' (amorce C),
5'-CTGTGGCCGATCAGGATGGTTTTGTCGTGC-3' (amorce D)

Une spécificité théorique de ces amorces est vérifiée vis-à-vis des banques de données complètes de Genbank et EMBL, pour une homologie (complète ou partielle) permettant jusqu'à 5 nucléotides différents (mismatch).

Amplification d'ADN: le protocole de PCR utilisé est dérivé de celui recommandé par Perkin-Elmer Cetus Corporation. La réaction a lieu dans un volume de 50 µl avec 50 µl d'huile minérale (Sigma).

Pour ce qui concerne *Ec,* le mélange est soumis à 25 cycles des incubations suivantes : 1 mn à 94°C, 1 mn à 65°C, 1 mn 30s à 72°C (Pharmacia LKB Gene ATAQ Controller DFAT 70-01-101).

Pour *Ech* ces 25 cycles sont effectués de la manière suivante: 1 mn à 94°C, 2 mn à 72°C. L'analyse initiale des produits de PCR est faite par electrophorèse sur des minigels d'agarose à 1% (p/v).

### Analyse des fragments de restriction :

Les produits de PCR sont précipités à l'ethanol et resuspendus dans 20µl de tampon TE (Tris EDTA) (Maniatis, 1982). Les enzymes de restriction utilisés dans le cas d'*Ec* sont *Alu*I, *Hae*II, *Sau*A1, *Hpa*II, *Taq*1, *Hae*III et *Hha*1 de Boehringer. La restriction a lieu dans un volume de 15 µl, avec 5 µl d'ADN pendant 1 ou 2 heures, à 37°C. Dans le cas d'*Ech* ces enzymes sont: *Sau*3A1, *Alu*I et *Hpa*II. La totalité du volume est soumise à une électrophorèse sur gel d'acrylamide à 6% (solution mère: 28% d'acrylamide, Appligène, 2% bis, Biorad), dans un tampon TBE (Tris Borate EDTA) (Maniatis, 1982) à 250 volts pendant 1 heure. Le gel est coloré par du bromure d'éthidium (2 mg/l), puis rincé. Un µg de marqueur V (Boehringer) et une échelle d'ADN de 1kb (BRL) sont utilisés comme marqueurs de taille.

### B) Résultats:

### Essai de PCR :

Les amorces A et B ont été expérimentées dans un premier temps pour 6 souches bactériennes pour leur spécificité avec des conditions de température variables.

Les meilleures conditions d'amplification déterminées (avec un appareil Pharmacia) sont: 94°C 1 min, 65°C 1 min, 72°C 1 min 30s, 25 cycles. Une collection de microorganismes est examinée pour la spécificité de ce protocole PCR (Voir tableau 1). Parmi cette collection, toutes les souches d'*Eca, Ecc, Eco* et *Ecw,* présentent un fragment amplifié de 434 pb. Aucune des souches *Ecb (Erwinia carotovora* subsp. *betavascularum), Ech* ou autres espèces d'*Erwinia* telles que *herbicola* ou *rhapontici* ne sont détectées. Les autres microorganismes expérimentés ne présentent pas de signal positif. Ces amorces, dans ces conditions de PCR, permettent la détection spécifique de quatre des actuelles sous-espèces d'*Erwinia carotovora* pectinolytiques, notamment sur la pomme de terre.

Les meilleures conditions d'amplification déterminées (avec un appareil Pharmacia) pour les amorces C et D sont les suivantes: 94°C 1 min et 72°C 2 min.

A l'aide des amorces C et D, les souches *Ech* sont détectées spécifiquement et ainsi des erwinias pathogènes sur les pommes de terre ont pu être détectées avec une discrimination possible entre les espèces *chrysanthemi* et *carotovora.* En raison des différences physiologiques, géographiques et du pouvoir pathogène des deux espèces, ces tests PCR peuvent jouer un rôle important, notamment pour la certification des semences et boutures.

Ceci étant, l'essai effectué sur *Erwinia carotovora* ne permet pas de différencier les souches les plus pathogènes sur pomme de terre dans les champs (principalement *Eca)* des autres souches responsables, entre autres, des maladies de stockage *(Ecc).* Pour cette raison, une étude RFLP sur la région amplifiée est entreprise pour rechercher un polymorphisme éventuellement corrélé au pouvoir pathogène, la taxonomie, la région géographique d'isolement.

### Analyse RFLP:

Les fragments amplifiés de 434 pb de la collection des souches *Ec* sont restreints avec plusieurs enzymes: *Taq*1 et *Hae*III ne donnent pas de polymorphisme lorsqu'ils sont testés sur une collection de souches variées. *Hha*1 donne plus de 15 profils sur une gamme de 30 souches représentatives des souches d'*Ec. Alu*I, *Hae*III, *Sau*3A1 et *Hpa*II donnent 3 à 5 profils différents (figure 3) pour la totalité de la collection (environ 100 souches). Les résultats globaux sont représentés au tableau 1. L'enzyme *Sau3*A1 permet l'identification des souches *Eca* isolées à partir de pomme de terre (profil IV de la figure 3), agents responsables chez cette dernière de la jambe noire typique. Deux souches d'*Eca* isolées de tomate ont le profil IV également, mais ces souches sont également responsables de symptômes de jambe noire sur tomate et pomme de terre lorsqu'elles sont inoculées (Samson et *al.,* 1989). Il pourrait s'agir de souches d'*Eca* spécifiques des pommes de terre et trouvées incidemment sur des tomates ou, peut être, n'existe-t-il de réelle différence dans le pouvoir pathogène d'*Eca* pour ces deux espèces végétales proches taxonomiquement.

Une exception est constituée par la souche 89.19 isolée de la pomme de terre en Argentine qui ne présente pas le profil IV. Cette souche appartient au profil II comme les souches *Eca,* 1H et 40H, atypiques isolées de l'eau en Espagne. Ces souches présentent en commun d'autres caractéristiques comme la faculté de croître à 37°C (les souches d'*Eca* typiques ne le font pas) et ne donnent pas les symptômes typiques de la jambe noire lorsqu'elles sont inoculées à des plants de pomme de terre. Ces souches ne semblent donc pas appartenir à la sous-espèce *atroseptica.*

Dans cet essai PCR, la restriction par *Sau*3A1 donne des résultats homogènes pour l'identification de l'agent typique de la jambe noire. D'un point de vue diagnostic, la seule utilisation du test PCR pourrait donner une bonne estimation des risques encourus au champ et en stockage. Les informations obtenues par l'utilisation de *Sau*3A1, et donc du polymorphisme indiqué par cette enzyme, permettent d'identifier spécifiquement les agents typiques de la jambe noire.

Les profils obtenus à l'aide des enzymes *Sau*3A1, *Hpa*II et *Alu*I à partir de gènes codant des PL chez *Ech,* permettent de distinguer des souches d'*Ech* en fonction de leurs hôtes d'origine, et plus particulièrement les pathovars, par exemple *zeae* et *dianthicola..*

Pour *Ech,* on notera également qu'il y a une relation entre le type de profil obtenu et la plante d'origine, et plus particulièrement le pathovar. Par exemple avec l'enzyme *Sau*3A1 permet d'identifier les souches spécifiques de l'oeillet, du maïs, du Saintpaulia, du Dieffenbachia, du Philodendron; l'enzyme *Alu*I permet d'identifier les souches du maïs.

Comme nous l'avons vu précédemment, les amorces A et B d'une part, et C et D d'autre part, sont respectivement spécifiques des *Ec* et des *Ech.* Il convient de noter que ces amorces ne permettent en aucun cas l'amplification d'*Erwinia herbicola, Erwinia rhapontici, Franckia, Pseudomonas* sp., *Xanthomonas campestris, Agrobacterium tumefaciens, Clavibacter* sp. (anciennement nommée *Corynebacterium),* des organismes fixateurs d'azote atmosphérique, des champignons et levures saprophytes de pomme de terre, et de *Yersinia* sp., certains de ces organismes produisant des pectate-lyases.

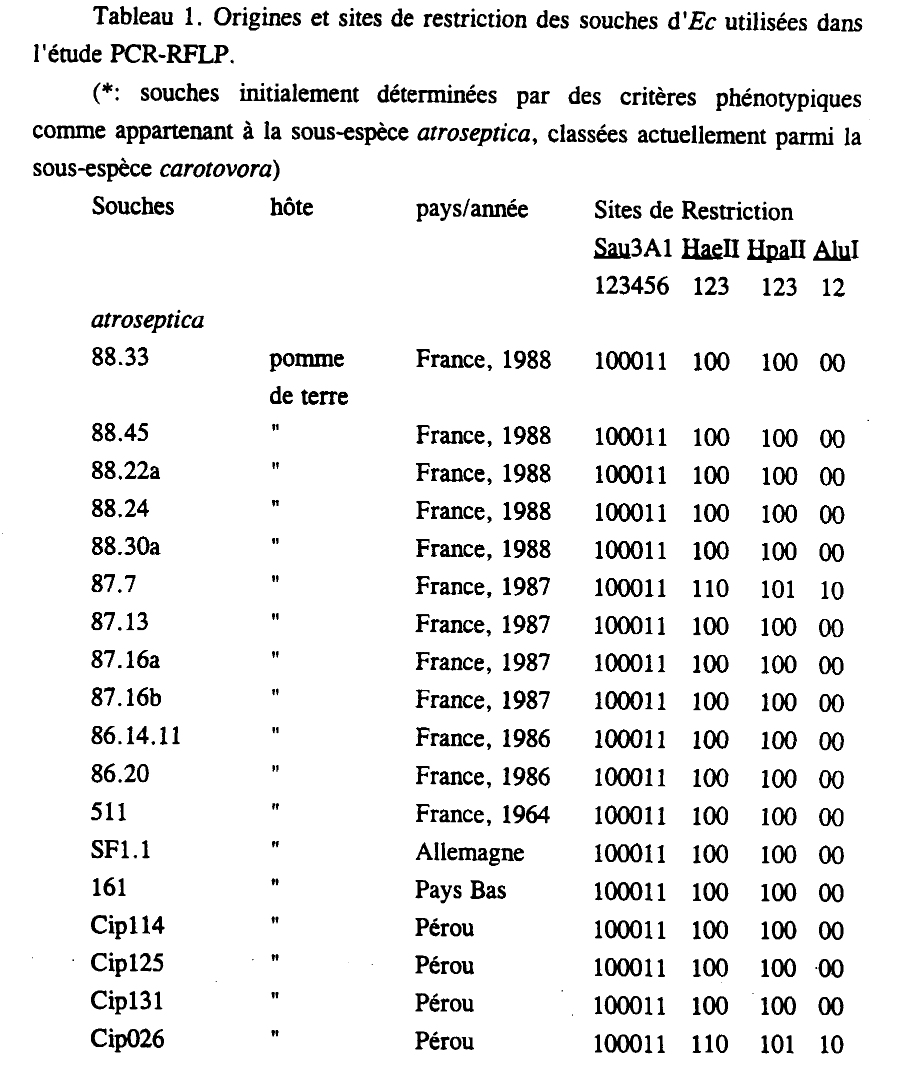

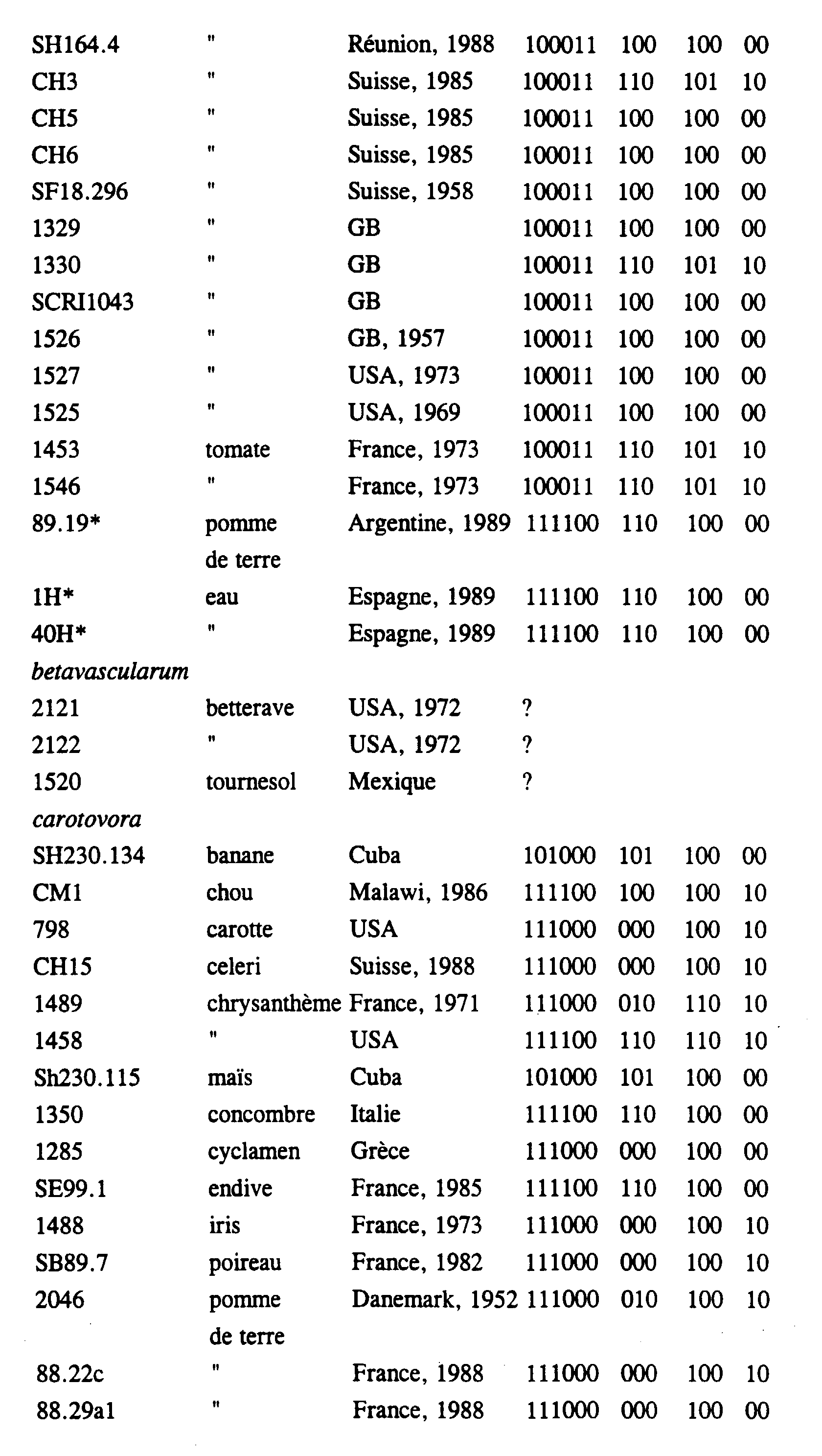

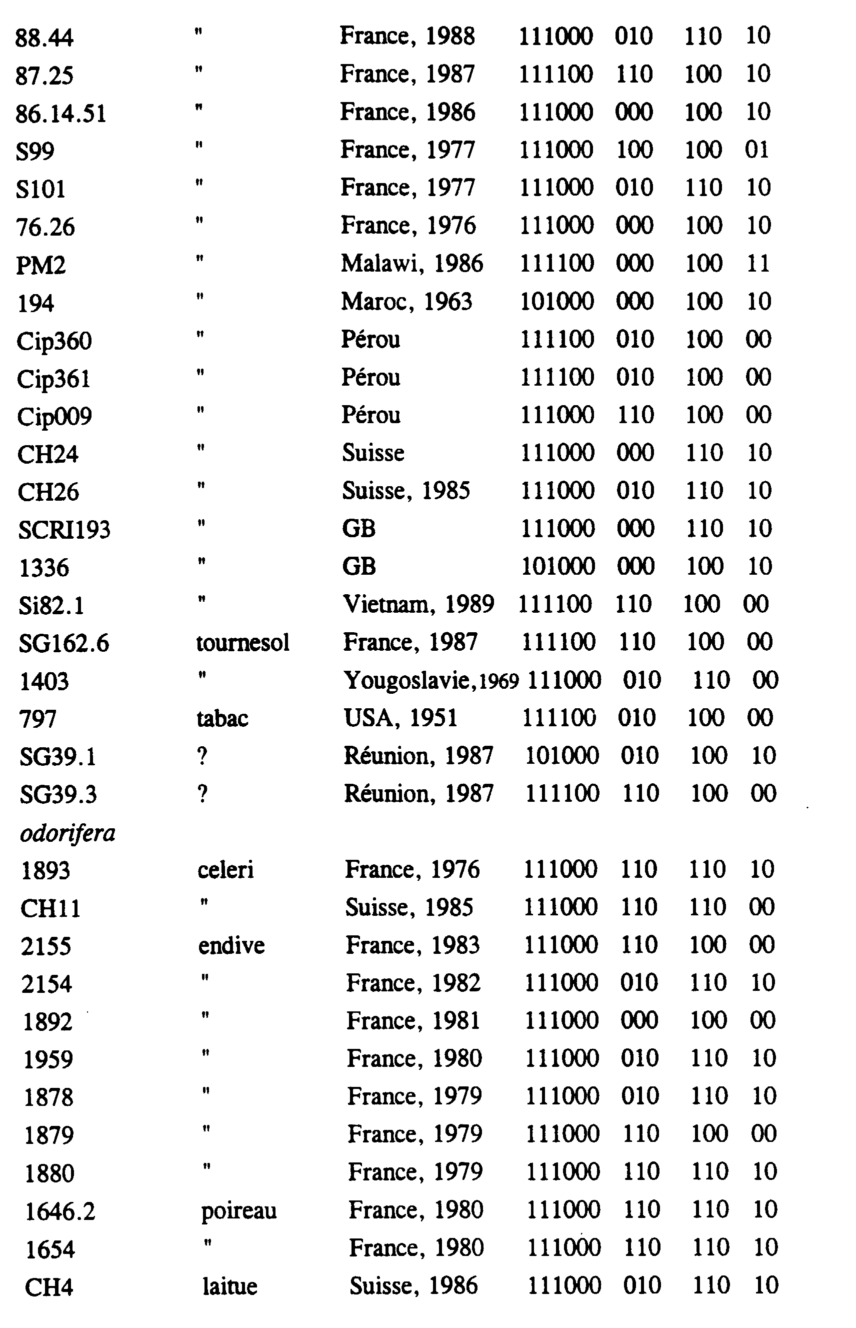

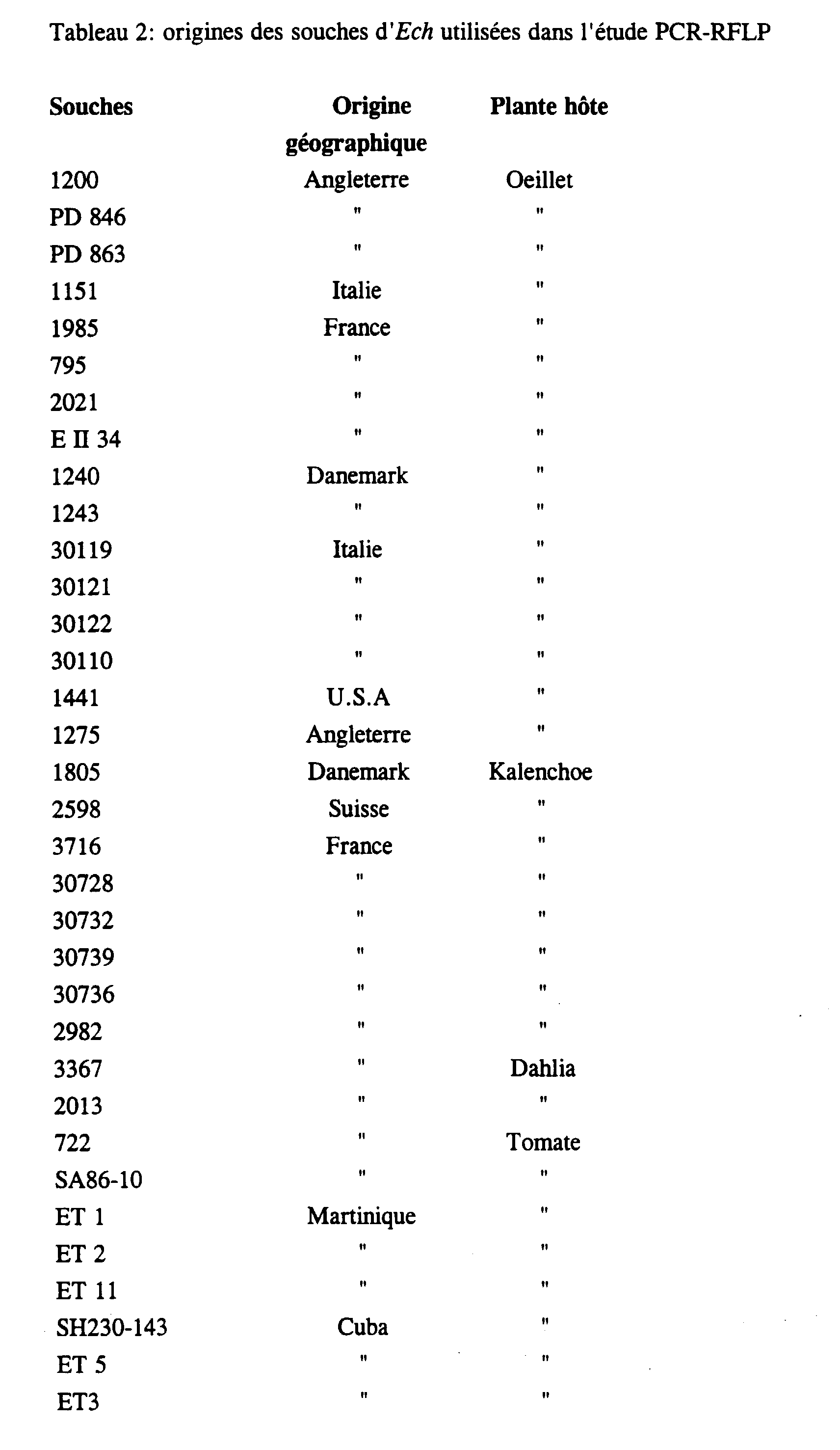

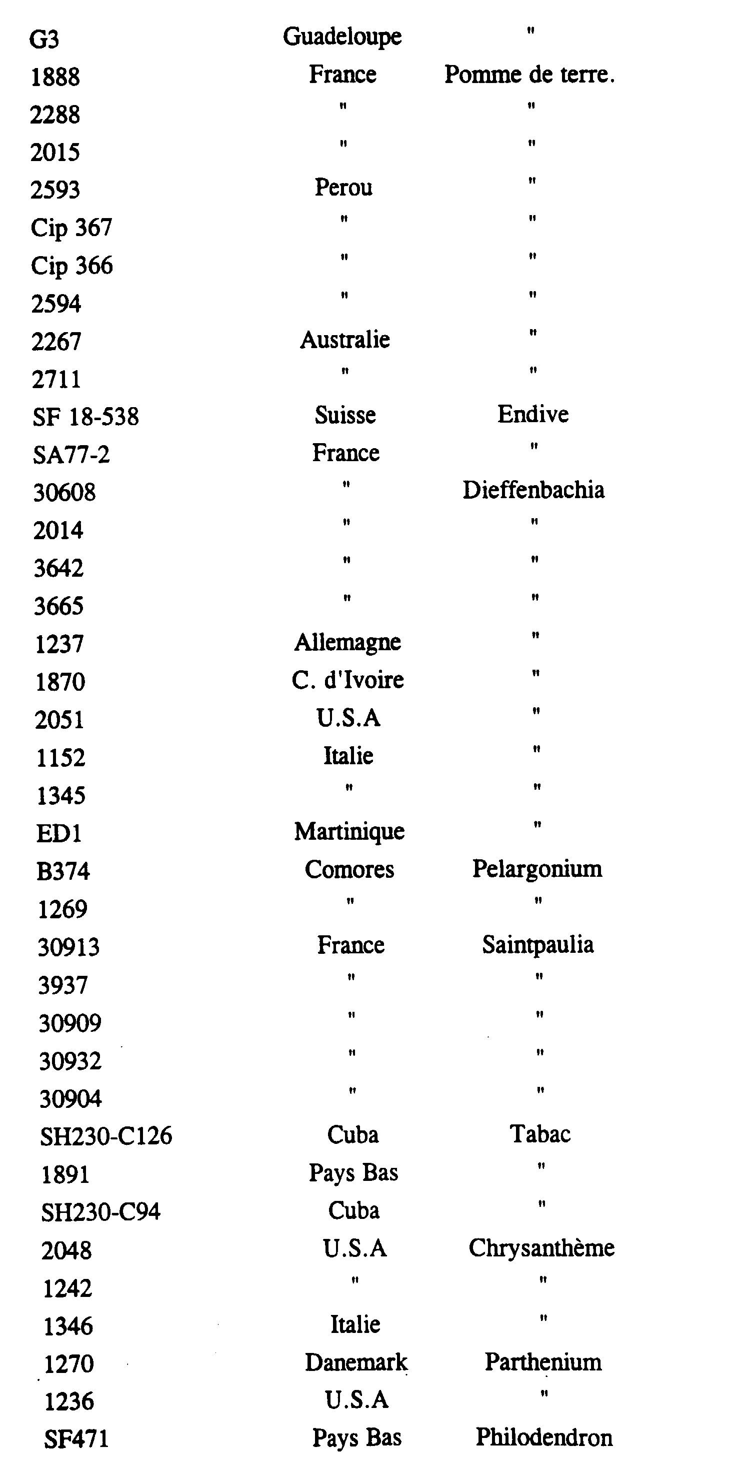

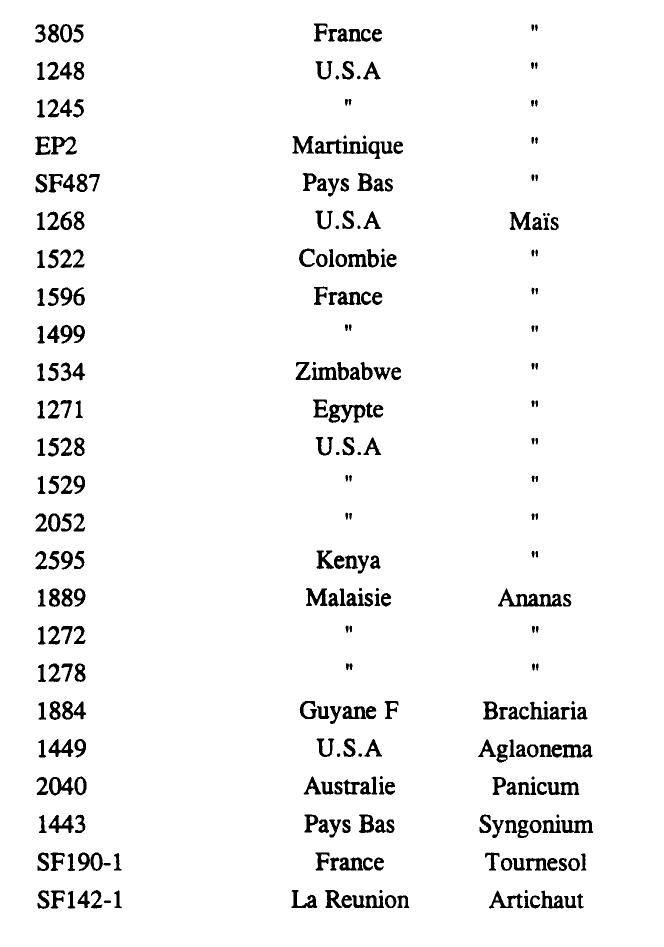

### BIBLIOGRAPHIE

- Barany, 1991, Biochemistry, Vol 30, n°11, 2735
- Bloch, 1991, Proc. Natl. Acad. Sci. USA, Vol 88, pp. 189- 193
- Favey *et al.,* 1992, Journal of General Microbiology, 138: 499-508
- Gilliland *et al.,* 1990, PCR Protocols. A Guide to Methods and Applications. Edited by Innis, Gelfand, Sninsky, White. Academic Press Inc
- Hinton *et al.,* 1989, Molecular Microbiology, 3: 1785-1795
- Ito *et al.,* 1988, Agric. Biol. Chem., 52: 479487
- klotz and Zimm, 1972, J. Mol. Biol., 72: 779-800
- Lei *et al.,* 1987, Journal of Bacteriology, 169: 4379-4383
- Lei et al., 1988, Gene, 62: 159-164
- Maniatis *et al.,* 1982, Molecular Cloning: A Laboratory Manual. Cold Spring Harbor Laboratory, Cold Spring Harbor, NY, 545 pp.
- Manulis, 1988, Journal of Bacteriology, 170: 1825-1830
- Miller, 1972, Experiments in Molecular Genetics. Cold Spring Harbor Laboratory, Cold Spring Harbor, NY, 466 pp.
- Samson *et al.,* 1989, Biochemical and serological diversity of Erwinia chrysanthemi, in Proc. 7th Int. Conf. Plant Path. Bact., Budapest, Hungary, pp: 895-900
- Schoedel and Collmer, 1986, Journal of Bacteriology, 167:117-123
- Trollinger *et al.,* 1989, Molecular Plant Microbe Interactions, 2: 17-25
- Van Gijsegem, 1989, Molecular Microbiology, 3: 1415-1424.

## Revendications (Revendications pour l'(les) Etat(s) contractant(s) suivant(s): AT, BE, CH, DE, DK, FR, GB, GR, IE, IT, LI, LU, MC, NL, PT, SE)

1. Procédé d'identification et de détection de bactéries pectinolytiques du genre *Erwinia* d'une espèce déterminée, et plus particulièrement de l'espèce *Erwinia chrysanthemi* et de l'espèce *Erwinia carotovora,* dans le sol ou l'eau. ou chez un hôte susceptible d'être porteur de telles bactéries, notamment chez les plantes et les semences, ce procédé consistant à rechercher les fragments d'ADN ou d'ARN, spécifiques de l'espèce, la sous-espèce ou du pathovar concernés, codant une pectate-lyase (PL), en mettant en jeu une hybridation moléculaire avec au moins une sonde spécifique, cette hybridation étant précédée par une amplification du nombre de copies des fragments d'ADN susmentionnés à l'aide d'amorces spécifiques.

2. Séquence nucléotidique caractérisée en ce qu'elle comprend tout ou partie de la séquence. nucléotidique A suivante: 5'-TTACCGGACGCCGAGCTGTGGCGT-3' ou sa séquence complémentaire, ou une séquence dérivée de la séquence A, notamment par suppression et/ou substitution et/ou addition de nucléotides, et susceptible de s'hybrider avec le fragment délimité par les positions 1231 et 1254 de la séquence représentée sur la figure 1, ou la séquence complémentaire de cette séquence dérivée.

3. Séquence nucléotidique caractérisée en ce qu'elle comprend tout ou partie de la séquence nucléotidique B choisie parmi tout ou partie:
* des enchaînements de nucléotides suivants: 
5'-CAGGAAGATGTCGTTATCGCGAGT-3'
..........C.............
..........T.............
 ou leurs séquences complémentaires,
* ou d'une séquence dérivée de la séquence B, notamment par suppression et/ou substitution et/ou addition de nucléotides, et susceptible de s'hybrider avec la séquence complémentaire du fragment délimité par les positions 1642 et 1665 de la séquence représentée sur la figure 1, ou la séquence complémentaire de cette séquence dérivée.

4. Séquence nucléotidique caractérisée en ce qu'elle comprend tout ou partie de la séquence nucléotidique C choisie parmi tout ou partie:
* des enchaînements de nucléotides suivants: 
5'-GATCAGAAAGCCCGCAGCCAGAT-3'
.....A........T........
...........G..T........
 (les points représentant des nucléotides identiques à ceux de la première ligne) ou leurs séquences complémentaires,
* ou d'une séquence dérivée de la séquence C, notamment par suppression et/ou substitution et/ou addition de nucléotides, et susceptible de s'hybrider avec le fragment délimité par les positions 295 et 317 de la séquence représentée sur la figure 2, ou la séquence complémentaire de cette séquence dérivée.

5. Séquence nucléotidique caractérisée en ce qu'elle comprend tout ou partie de la séquence nucléotidique D choisie parmi tout ou partie:
* des enchaînements de nucléotides suivants: 
5'-CTGTGGCCGATCAGGATGGTTTTGTCGTGC-3'
..A.......................A...
T.............C...............
.....A..A.....C...............
.................A............
 ou leurs séquences complémentaires,
* ou d'une séquence dérivée de la séquence D, notamment par suppression et/ou substitution et/ou addition de nucléotides, et susceptible de s'hybrider avec la séquence complémentaire du fragment délimité par les positions 672 et 701 de la séquence représentée sur la figure 2, ou la séquence complémentaire de cette séquence dérivée.

6. Couples d'amorces pour l'amplification génique, caractérisés en ce que l'une des amorces de ces couples est choisie parmi les séquences comprenant tout ou partie de la séquence nucléotidique A, ou une séquence dérivée, selon la revendication 2, tandis que l'autre amorce est choisie parmi les séquences comprenant tout ou partie de la séquence nucléotidique B, ou une séquence dérivée, selon la revendication 3.

7. Couples d'amorces pour l'amplification génique, caractérisés en ce que l'une des amorces de ces couples est choisie parmi les séquences comprenant tout ou partie de la séquence nucléotidique C, ou une séquence dérivée, selon la revendication 4 tandis que l'autre amorce est choisie parmi les séquences comprenant tout ou partie de la séquence nucléotidique D, ou une séquence dérivée, selon la revendication 5.

8. Sonde nucléotidique caractérisée en ce qu'elle comprend au moins 10 nucléotides de la séquence délimitée par les nucléotides situés aux positions 1231 et 1665 de la séquence représentée sur la figure 1, ou au moins 10 nucléotides ayant au minimum 40% d'homologie avec la séquence susmentionnée.

9. Sonde nucléotidique caractérisée en ce qu'elle comprend au moins 10 nucléotides de la séquence délimitée par les nucléotides situés aux positions 295 et 701 de la séquence représentée sur la figure 2, ou au moins 10 nucléotides ayant au minimum 40% d'homologie avec la séquence susmentionnée.

10. Séquences nucléotidiques selon l'une des revendications 2 à 9, caractérisées en ce qu'elles sont marquées, notamment de manière radioactive, enzymatique, par un composé fluorescent, par liaison à une molécule antigènique (susceptible d'être reconnue par des anticorps) ou à toute autre molécule susceptible d'être directement ou indirectement détectée à l'aide de réactifs, cette liaison pouvant être effectuée par l'intermédiaire d'un bras espaceur, notamment par l'intermédiaire d'une séquence nucléotidique constituée d'environ 5 à 100 nucléotides située aux extrémités 3' ou 5' de ces séquences.

11. Procédé d'identification et de détection selon la revendication 1, d'*Erwinia carotovora* dans le sol ou l'eau, ou chez un hôte, notamment chez les plantes et les semences, ce procédé comprenant les étapes suivantes:
- le traitement d'un échantillon prélevé dans l'eau, le sol ou chez cet hôte, de manière à rendre le génome d'*Erwinia carotovora* accessible aux amorces définies dans la revendication 6, et le cas échéant à toute ADN ou ARN polymérase permettant de répliquer les deux brins de l'ADN génomique ou l'ARN en dérivant, ce traitement étant notamment effectué par ébullition, macération (dans un liquide tel que l'eau), broyage, ou sonication,
- l'amplification du nombre de copies de fragments de gènes codant des PL, susceptibles d'être présents dans cet échantillon, à l'aide de couples d'amorces tels que définis ci-dessus,
- la détection de la présence éventuelle de gènes codant des PL, et donc de la présence d'*Erwinia carotovora* dans l'échantillon étudié, à l'aide d'une sonde selon la revendication 8, le cas échéant marquée, notamment de la manière indiquée dans la revendication 10.

12. Procédé d'identification et de détection selon la revendication 1, d'*Erwinia chrysanthemi* dans le sol ou l'eau, ou chez un hôte, notamment chez les plantes et les semences, ce procédé comprenant les étapes suivantes:
- le traitement d'un échantillon prélevé dans l'eau, le sol ou sur un hôte, de manière à rendre le génome des *Erwinia chrysanthemi* accessible aux amorces définies dans la revendication 7, et le cas échéant à toute ADN ou ARN polymérase permettant de répliquer les deux brins de l'ADN génomique ou l'ARN en dérivant, ce traitement étant notamment effectué par ébullition, macération (dans un liquide tel que l'eau), broyage, ou sonication,
- l'amplification du nombre de copies de fragments de gènes codant des PL, susceptibles d'être présents dans cet échantillon, à l'aide de couples d'amorces tels que définis ci-dessus,
- la détection de la présence éventuelle de gènes codant des PL, et donc de la présence d'*Erwinia chrysanthemi* dans l'échantillon étudié à l'aide d'une sonde selon la revendication 9, le cas échéant marquée, notamment de la manière indiquée dans la revendication 10.

13. Procédé selon la revendication 11 ou 12, caractérisé en ce que l'amplification du nombre de gènes codant des pectate-lyases comprend les étapes suivantes:
- la prédénaturation de l'ADN double brin en ADN mono-brin, de préférence dans un tampon constitué de Tris-HCl 10 mM pH 8,3, 50 mM KCl, 1,5 mM MgCl₂, 0,01% de gélatine, de cofacteurs de l'ADN (ou ARN)-polymérase, notamment d'ions Mg^{2 +} et K⁺, des 4 désoxynucléotides constitutifs des ADN (dCTP, dATP, dGTP, dTTP), ou des ARN (dCTP, dUTP, dGTP, dTTP), et des couples d'amorces tels que définis dans la revendication 6 ou 7, par chauffage entre environ 80°C et environ 110°C, avantageusement à 100°C,
- l'amplification proprement dite par addition au milieu obtenu à l'étape précédente d'ADN polymérase thermorésistante par exemple, et
* chauffage à environ 94°C, ce qui correspond à l'étape de dénaturation proprement dite,
* puis chauffage entre environ 60°C et environ 76°C, ce qui correspond à l'étape d'hybridation des couples d'amorces avec les gènes codant des PL, ou des fragments de ces gènes, susceptibles d'être présents dans l'échantillon biologique étudié,
* et enfin chauffage entre environ 50°C et environ 76°C, ce qui correspond à l'étape d'élongation des amorces, hybridées à l'étape précédente, l'une vers l'autre, produisant ainsi des séquences nucléotidiques complémentaires de tout ou partie des séquences génomiques des erwinias pectinolytiques, ces dernières séquences étant délimitées par les nucléotides s'hybridant avec les amorces susmentionnées,
- la répétition de l'étape d'amplification précédente entre environ 20 et environ 50 fois, avantageusement entre 25 et 35 fois.
- le cas échéant, la récupération d'une partie aliquote du milieu obtenu à la fin de l'étape d'amplification précédente, afin de réaliser une nouvelle amplification selon la méthode décrite ci-dessus.

14. Application du procédé selon l'une des revendications 11 à 13, à la mise en oeuvre d'une méthode de diagnostic de l'éventuelle apparition de pathologies causées par les bactéries de l'espèce *Erwinia carotovora et* plus particulièrement par l'une des sous-espèces *Ecc, Eca, Eco* et *Ecw,* par les bactéries de l'espèce *Erwinia chrysanthemi,* ou de toute espèce, sous-espèce ou pathovar, actuels ou dérivés ultérieurement par modification de la taxonomie actuelle.

15. Kit pour la mise en oeuvre d'un procédé selon l'une des revendications 11 à 13, caractérisé en ce qu'il comprend:
- au moins un couple d'amorces selon la revendication 6, et/ou
- au moins un couple d'amorces selon la revendication 7,
- une (ou plusieurs) sonde(s) selon la revendication 8, et/ou une (ou plusieurs) sonde(s) selon la revendication 9, lesdites sondes étant susceptibles de s'hybrider avec tout ou partie des fragments de gènes amplifiés à l'aide des couples d'amorces selon les revendications 6 et 7 respectivement.

16. Kit selon la revendication 15, caractérisé en ce qu'il comprend également:
- une ADN ou ARN polymérase thermorésistante,
- un milieu réactionnel avantageusement constitué de Tris-HCl 10 mM pH 8,3, 50 mM KCl, 1,5 mM MgCl₂, 0,01% de gélatine, de cofacteurs de l'ADN-polymérase, notamment d'ions Mg²⁺ et K⁺, et des 4 désoxynucléotides constitutifs des ADN (dCTP, dATP, dGTP, dTTP) ou des ARN (dCTP, dATP, dGTP, dUTP) et/ou,
- une ou plusieurs enzymes de restriction, et, le cas échéant, des fragments de restriction de référence caractéristiques des sous-espèces d'*Erwinia carotovora* et/ou d'*Erwinia chrysanthemi,* notamment des fragments de restriction représentés sur la figure 3, dans le cas des sous-espèces d'*Erwinia carotovora* et/ou,
- une ligase et un (ou plusieurs) couple(s) de séquences oligonucléotidiques, susceptibles de s'hybrider de part et d'autre d'un (ou plusieurs) nucléotide(s) situé(s) dans des gènes ou fragment de gènes codant des pectate-lyases, et étant caractéristique(s) d'une espèce, sous-espèce ou pathovar d'*Erwinia,* et/ou
- une (ou plusieurs) sonde(s) oligonucléotidique(s) spécifique(s) d'une région nucléotidique caractéristique d'une espèce, sous-espèce ou pathovar déterminé de bactéries du genre *Erwinia.*

17. Utilisation de séquences nucléotidiques correspondant à tout ou partie de gènes qui, au sein des génomes des bactéries pectinolytiques du genre *Erwinia,* et plus particulièrement d'*Erwinia carotovora* et d'*Erwinia chrysanthemi,* codent des pectate-lyases, pour la mise en oeuvre d'un procédé d'identification des sous-espèces, et/ou des souches ou pathovars des espèces ou sous-espèces, des différentes bactéries du genre *Erwinia.*

## Revendications (Revendications pour l'(les) Etat(s) contractant(s) suivant(s): ES)

1. Procédé d'identification et de détection de bactéries pectinolytiques du genre *Erwinia* d'une espèce déterminée, et plus particulièrement de l'espèce *Erwinia chrysanthemi* et de l'espèce *Erwinia carotovora,* dans le sol ou l'eau, ou chez un hôte susceptible d'être porteur de telles bactéries, notamment chez les plantes et les semences, ce procédé consistant à rechercher les fragments d'ADN ou d'ARN, spécifiques de l'espèce, la sous-espèce ou du pathovar concernés, codant une pectate-lyase (PL), en mettant en jeu une hybridation moléculaire avec au moins une sonde spécifique, cette hybridation étant précédée par une amplification du nombre de copies des fragments d'ADN susmentionnés à l'aide d'amorces spécifiques.

2. Séquence nucléotidique caractérisée en ce qu'elle comprend tout ou partie de la séquence nucléotidique A suivante: 5'-TTACCGGACGCCGAGCTGTGGCGT-3' ou sa séquence complémentaire, ou une séquence dérivée de la séquence A, notamment par suppression et/ou substitution et/ou addition de nucléotides, et susceptible de s'hybrider avec le fragment délimité par les positions 1231 et 1254 de la séquence représentée sur la figure 1, ou la séquence complémentaire de cette séquence dérivée.

3. Séquence nucléotidique caractérisée en ce qu'elle comprend tout ou partie de la séquence nucléotidique B choisie parmi tout ou partie:
* des enchaînements de nucléotides suivants: 
5'-CAGGAAGATGTCGTTATCGCGAGT-3'
........C...............
........T...............
 ou leurs séquences complémentaires,
* ou d'une séquence dérivée de la séquence B, notamment par suppression et/ou substitution et/ou addition de nucléotides, et susceptible de s'hybrider avec la séquence complémentaire du fragment délimité par les positions 1642 et 1665 de la séquence représentée sur la figure 1, ou la séquence complémentaire de cette séquence dérivée.

4. Séquence nucléotidique caractérisée en ce qu'elle comprend tout ou partie de la séquence nucléotidique C choisie parmi tout ou partie:
* des enchaînements de nucléotides suivants: 
5'-GATCAGAAAGCCCGCAGCCAGAT-3'
.....A........T........
...........G..T........
 (les points représentant des nucléotides identiques à ceux de la première ligne) ou leurs séquences complémentaires,
* ou d'une séquence dérivée de la séquence C, notamment par suppression et/ou substitution et/ou addition de nucléotides, et susceptible de s'hybrider avec le fragment délimité par les positions 295 et 317 de la séquence représentée sur la figure 2, ou la séquence complémentaire de cette séquence dérivée.

5. Séquence nucléotidique caractérisée en ce qu'elle comprend tout ou partie de la séquence nucléotidique D choisie parmi tout ou partie:
* des enchaînements de nucléotides suivants: 
5'-CTGTGGCCGATCAGGATGGTTTTGTCGTGC-3'
..A.......................A...
T.............C...............
.....A..A.....C...............
.................A............
 ou leurs séquences complémentaires,
* ou d'une séquence dérivée de la séquence D, notamment par suppression et/ou substitution et/ou addition de nucléotides, et susceptible de s'hybrider avec la séquence complémentaire du fragment délimité par les positions 672 et 701 de la séquence représentée sur la figure 2, ou la séquence complémentaire de cette séquence dérivée.

6. Couples d'amorces pour l'amplification génique, caractérisés en ce que l'une des amorces de ces couples est choisie parmi les séquences comprenant tout ou partie de la séquence nucléotidique A, ou une séquence dérivée, selon la revendication 2, tandis que l'autre amorce est choisie parmi les séquences comprenant tout ou partie de la séquence nucléotidique B, ou une séquence dérivée, selon la revendication 3.

7. Couples d'amorces pour l'amplification génique, caractérisés en ce que l'une des amorces de ces couples est choisie parmi les séquences comprenant tout ou partie de la séquence nucléotidique C, ou une séquence dérivée, selon la revendication 4 tandis que l'autre amorce est choisie parmi les séquences comprenant tout ou partie de la séquence nucléotidique D, ou une séquence dérivée, selon la revendication 5.

8. Sonde nucléotidique caractérisée en ce qu'elle comprend au moins 10 nucléotides de la séquence délimitée par les nucléotides situés aux positions 1231 et 1665 de la séquence représentée sur la figure 1, ou au moins 10 nucléotides ayant au minimum 40% d'homologie avec la séquence susmentionnée.

9. Sonde nucléotidique caractérisée en ce qu'elle comprend au moins 10 nucléotides de la séquence délimitée par les nucléotides situés aux positions 295 et 701 de la séquence représentée sur la figure 2, ou au moins 10 nucléotides ayant au minimum 40% d'homologie avec la séquence susmentionnée.

10. Séquences nucléotidiques selon l'une des revendications 2 à 9, caractérisées en ce qu'elles sont marquées, notamment de manière radioactive, enzymatique, par un composé fluorescent, par liaison à une molécule antigènique (susceptible d'être reconnue par des anticorps) ou à toute autre molécule susceptible d'être directement ou indirectement détectée à l'aide de réactifs, cette liaison pouvant être effectuée par l'intermédiaire d'un bras espaceur, notamment par l'intermédiaire d'une séquence nucléotidique constituée d'environ 5 à 100 nucléotides située aux extrémités 3' ou 5' de ces séquences.

11. Procédé d'identification et de détection selon la revendication 1, d'*Erwinia carotovora* dans le sol ou l'eau, ou chez un hôte, notamment chez les plantes et les semences, ce procédé comprenant les étapes suivantes:
- le traitement d'un échantillon prélevé dans l'eau, le sol ou chez cet hôte, de manière à rendre le génome d'*Erwinia carotovora* accessible aux amorces définies dans la revendication 6, et le cas échéant à toute ADN ou ARN polymérase permettant de répliquer les deux brins de l'ADN génomique ou l'ARN en dérivant, ce traitement étant notamment effectué par ébullition, macération (dans un liquide tel que l'eau), broyage, ou sonication,
- l'amplification du nombre de copies -de fragments de gènes codant des PL, susceptibles d'être présents dans cet échantillon, à l'aide de couples d'amorces tels que définis ci-dessus,
- la détection de la présence éventuelle de gènes codant des PL, et donc de la présence d'*Erwinia carotovora* dans l'échantillon étudié, à l'aide d'une sonde selon la revendication 8, le cas échéant marquée, notamment de la manière indiquée dans la revendication 10.

12. Procédé d'identification et de détection selon la revendication 1, *d'Erwinia chrysanthemi* dans le sol ou l'eau, ou chez un hôte, notamment chez les plantes et les semences, ce procédé comprenant les étapes suivantes:
- le traitement d'un échantillon prélevé dans l'eau, le sol ou sur un hôte, de manière à rendre le génome des *Erwinia chrysanthemi* accessible aux amorces définies dans la revendication 7, et le cas échéant à toute ADN ou ARN polymérase permettant de répliquer les deux brins de l'ADN génomique ou l'ARN en dérivant, ce traitement étant notamment effectué par ébullition, macération (dans un liquide tel que l'eau), broyage, ou sonication,
- l'amplification du nombre de copies de fragments de gènes codant des PL, susceptibles d'être présents dans cet échantillon, à l'aide de couples d'amorces tels que définis ci-dessus,
- la détection de la présence éventuelle de gènes codant des PL, et donc de la présence d'*Erwinia chrysanthemi* dans l'échantillon étudié à l'aide d'une sonde selon la revendication 9, le cas échéant marquée, notamment de la manière indiquée dans la revendication 10.

13. Procédé selon la revendication 11 ou 12, caractérisé en ce que l'amplification du nombre de gènes codant des pectate-lyases comprend les étapes suivantes:
- la prédénaturation de l'ADN double brin en ADN mono-brin, de préférence dans un tampon constitué de Tris-HCl 10 mM pH 8,3, 50 mM KCl, 1,5 mM MgCl₂, 0,01 % de gélatine, de cofacteurs de l'ADN (ou ARN)-polymérase, notamment d'ions Mg²⁺ et K⁺, des 4 désoxynucléotides constitutifs des ADN (dCTP, dATP, dGTP, dTTP), ou des ARN (dCTP, dUTP, dGTP, dTTP), et des couples d'amorces tels que définis dans la revendication 6 ou 7, par chauffage entre environ 80°C et environ 110°C, avantageusement à 100°C,
- l'amplification proprement dite par addition au milieu obtenu à l'étape précédente d'ADN polymérase thermorésistante par exemple, et
* chauffage à environ 94°C, ce qui correspond à l'étape de dénaturation proprement dite,
* puis chauffage entre environ 60°C et environ 76°C, ce qui correspond à l'étape d'hybridation des couples d'amorces avec les gènes codant des PL, ou des fragments de ces gènes, susceptibles d'être présents dans l'échantillon biologique étudié,
* et enfin chauffage entre environ 50°C et environ 76°C, ce qui correspond à l'étape d'élongation des amorces, hybridées à l'étape précédente, l'une vers l'autre, produisant ainsi des séquences nucléotidiques complémentaires de tout ou partie des séquences génomiques des erwinias pectinolytiques, ces dernières séquences étant délimitées par les nucléotides s'hybridant avec les amorces susmentionnées,
- la répétition de l'étape d'amplification précédente entre environ 20 et environ 50 fois, avantageusement entre 25 et 35 fois.
- le cas échéant, la récupération d'une partie aliquote du milieu obtenu à la fin de l'étape d'amplification précédente, afin de réaliser une nouvelle amplification selon la méthode décrite ci-dessus.

14. Application du procédé selon l'une des revendications 11 à 13, à la mise en oeuvre d'une méthode de diagnostic de l'éventuelle apparition de pathologies causées par les bactéries de l'espèce *Erwinia carotovora et* plus particulièrement par l'une des sous-espèces *Ecc, Eca, Eco* et *Ecw,* par les bactéries de l'espèce *Erwinia chrysanthemi,* ou de toute espèce, sous-espèce ou pathovar, actuels ou dérivés ultérieurement par modification de la taxonomie actuelle.

15. Kit pour la mise en oeuvre d'un procédé selon l'une des revendications 11 à 13, caractérisé en ce qu'il comprend:
- au moins un couple d'amorces selon la revendication 6, et/ou
- au moins un couple d'amorces selon la revendication 7,
- une (ou plusieurs) sonde(s) selon la revendication 8, et/ou une (ou plusieurs) sonde(s) selon la revendication 9, lesdites sondes étant susceptibles de s'hybrider avec tout ou partie des fragments de gènes amplifiés à l'aide des couples d'amorces selon les revendications 6 et 7 respectivement.

16. Kit selon la revendication 15, caractérisé en ce qu'il comprend également:
- une ADN ou ARN polymérase thermorésistante,
- un milieu réactionnel avantageusement constitué de Tris-HCl 10 mM pH 8,3, 50 mM KCl, 1,5 mM MgCl₂, 0,01% de gélatine, de cofacteurs de l'ADN-polymérase, notamment d'ions Mg²⁺ et K⁺, et des 4 désoxynucléotides constitutifs des ADN (dCTP, dATP, dGTP, dTTP) ou des ARN (dCTP, dATP, dGTP, dUTP) et/ou,
- une ou plusieurs enzymes de restriction, et, le cas échéant, des fragments de restriction de référence caractéristiques des sous-espèces d'*Erwinia carotovora* et/ou d'*Erwinia chrysanthemi,* notamment des fragments de restriction représentés sur la figure 3, dans le cas des sous-espèces d'*Erwinia carotovora* et/ou,
- une ligase et un (ou plusieurs) couple(s) de séquences oligonucléotidiques, susceptibles de s'hybrider de part et d'autre d'un (ou plusieurs) nucléotide(s) situé(s) dans des gènes ou fragment de gènes codant des pectate-lyases, et étant caractéristique(s) d'une espèce, sous-espèce ou pathovar d'*Erwinia,* et/ou
- une (ou plusieurs) sonde(s) oligonucléotidique(s) spécifique(s) d'une région nucléotidique caractéristique d'une espèce, sous-espèce ou pathovar déterminé de bactéries du genre *Erwinia.*

17. Utilisation de séquences nucléotidiques correspondant à tout ou partie de gènes qui, au sein des génomes des bactéries pectinolytiques du genre *Erwinia,* et plus particulièrement d'*Erwinia carotovora* et d'*Erwinia chrysanthemi,* codent des pectate-lyases, pour la mise en oeuvre d'un procédé d'identification des sous-espèces, et/ou des souches ou pathovars des espèces ou sous-espèces, des différentes bactéries du genre *Erwinia.*

18. Procédé de préparation d'une séquence nucléotidique comprenant tout ou partie de la séquence nucléotidique A suivante : 5'- TTACCGGACGCCGAGCTGTGGCGT-3' ou sa séquence complémentaire, ou une séquence dérivée de la séquence A, notamment par suppression et/ou substitution et/ou addition de nucléotides, et susceptible de s'hybrider avec le fragment délimité par les positions 1231 et 1254 de la séquence représentée sur la figure 1, ou la séquence complémentaire de cette séquence dérivée, ledit procédé étant effectué soit par voie biologique, notamment après transformation et clonage d'hôtes cellulaires susceptibles de contenir cette séquence nucléotidique, ou par amplification du nombre de copies de cette séquence nucléotidique, notamment selon la technique PCR, soit par voie chimique, notamment par la méthode automatisée β-cyanéthyl phosphoramidite décrite dans Bioorganic Chemisty 4 ; 274 - 325, 1986, ou par la méthode d'assemblage, par exemple par aboutage (ou ligation, par action d'une ligase), d'oligonucléotides synthétisés chimiquement, pourvus à leurs extrémités de sites de restriction adéquats, selon le principe décrit dans Proc. Nat. Acad. Sci. USA, 80 ; 7461 - 7465, 1983, ces méthodes étant suivies par le clonage des ADN ainsi obtenus dans un vecteur, notamment plasmidique, approprié et la récupération des ADN par hybridation avec une sonde appropriée, ou par électrophorèse, généralement après restriction, suivie d'une élution des fragments.

19. Procédé de préparation d'une séquence nucléotidique comprenant tout ou partie de la séquence nucléotidique B choisie parmi tout ou partie:
* des enchaînements de nucléotides suivants: 
5'-CAGGAAGATGTCGTTATCGCGAGT-3'
..........C.............
..........T.............
 ou leurs séquences complémentaires,
* ou d'une séquence dérivée de la séquence B, notamment par suppression et/ou substitution et/ou addition de nucléotides, et susceptible de s'hybrider avec la séquence complémentaire du fragment délimité par les positions 1642 et 1665 de la séquence représentée sur la figure 1, ou la séquence complémentaire de cette séquence dérivée, ledit procédé étant effectué selon les méthodes décrites dans la revendication 18.

20. Procédé de préparation d'une séquence nucléotidique comprenant tout ou partie de la séquence nucléotidique C choisie parmi tout ou partie:
* des enchaînements de nucléotides suivants: 
5'-GATCAGAAAGCCCGCAGCCAGAT-3'
.....A.......T........
..........G..T........
 (les points représentant des nucléotides identiques à ceux de la première ligne) ou leurs séquences complémentaires,
* ou d'une séquence dérivée de la séquence C, notamment par suppression et/ou substitution et/ou addition de nucléotides, et susceptible de s'hybrider avec le fragment délimité par les positions 295 et 317 de la séquence représentée sur la figure 2, ou la séquence complémentaire de cette séquence dérivée, ledit procédé étant effectué selon les méthodes décrites dans la revendication 18.

21. Procédé de préparation d'une séquence nucléotidique comprenant tout ou partie de la séquence nucléotidique D choisie parmi tout ou partie:
* des enchaînements de nucléotides suivants: 
5'-CTGTGGCCGATCAGGATGGTTTTGTCGTGC-3'
..A........................A..
T..............C..............
.....A..A......C..............
..................A...........
 ou leurs séquences complémentaires,
* ou d'une séquence dérivée de la séquence D, notamment par suppression et/ou substitution et/ou addition de nucléotides, et susceptible de s'hybrider avec la séquence complémentaire du fragment délimité par les positions 672 et 701 de la séquence représentée sur la figure 2, ou la séquence complémentaire de cette séquence dérivée, ledit procédé étant effectué selon les méthodes décrites dans la revendication 18.

22. Procédé de préparation de couples d'amorces pour l'amplification génique, l'une des amorces de ce couple étant choisie parmi les séquences comprenant tout ou partie de la séquence nucléotidique A, ou une séquence dérivée, selon la revendication 18, tandis que l'autre amorce est choisie parmi les séquences comprenant tout ou partie de la séquence nucléotidique B, ou une séquence dérivée, selon la revendication 19, ledit procédé étant effectué selon les méthodes décrites dans la revendication 18.

23. Procédé de préparation de couples d'amorces pour l'amplification génique, l'une des amorces de ce couple étant choisie parmi les séquences comprenant tout ou partie de la séquence nucléotidique C, ou une séquence dérivée, selon la revendication 20 tandis que l'autre amorce est choisie parmi les séquences comprenant tout ou partie de la séquence nucléotidique D, ou une séquence dérivée, selon la revendication 21, ledit procédé étant effectué selon les méthodes décrites dans la revendication 18.

24. Procédé de préparation d'une sonde nucléotidique comprenant au moins 10 nucléotides de la séquence délimitée par les nucléotides situés aux positions 1231 et 1665 de la séquence représentée sur la figure 1, ou au moins 10 nucléotides ayant au minimum 40% d'homologie avec la séquence susmentionnée, ledit procédé étant effectué selon les méthodes décrites dans la revendication 18.

25. Procédé de préparation d'une sonde nucléotidique comprenant au moins 10 nucléotides de la séquence délimitée par les nucléotides situés aux positions 295 et 701 de la séquence représentée sur la figure 2, ou au moins 10 nucléotides ayant au minimum 40% d'homologie avec la séquence susmentionnée, ledit procédé étant effectué selon les méthodes décrites dans la revendication 18.

26. Procédé de préparation de séquences nucléotidiques telle que définies dans l'une des revendications 18 à 25, marquées, notamment de manière radioactive, enzymatique, par un composé fluorescent, par liaison à une molécule antigènique (susceptible d'être reconnue par des anticorps) ou à toute autre molécule susceptible d'être directement ou indirectement détectée à l'aide de réactifs, cette liaison pouvant être effectuée par l'intermédiaire d'un bras espaceur, notamment par l'intermédiaire d'une séquence nucléotidique constituée d'environ 5 à 100 nucléotides située aux extrémités 3' ou 5' de ces séquences, ledit procédé étant effectué selon les méthodes décrites dans la revendication 18.

27. Procédé de préparation d'un kit pour la mise en oeuvre d'un procédé selon l'une des revendications 11 à 13, ledit procédé comprenant l'étape consistant à rassembler :
- au moins un couple d'amorces tel que défini dans la revendication 22, et/ou
- au moins un couple d'amorces tel que défini dans la revendication 23,
- une (ou plusieurs) sonde(s) telle(s) que définie(s) dans la revendication 24, et/ou une (ou plusieurs) sonde(s) telle(s) que définie(s) dans la revendication 25, lesdites sondes étant susceptibles de s'hybrider avec tout ou partie des fragments de gènes amplifiés à l'aide des couples d'amorces tels que définis dans les revendications 22 et 23 respectivement.

28. Procédé de préparation d'un kit selon la revendication 27, ledit procédé comprenant également l'étape consistant à rassembler :
- une ADN ou ARN polymérase thermorésistante,
- un milieu réactionnel avantageusement constitué de Tris-HCl 10 mM pH 8,3, 50 mM KCl, 1,5 mM MgCl₂, 0,01% de gélatine, de cofacteurs de l'ADN-polymérase, notamment d'ions Mg²⁺ et K⁺, et des 4 désoxynucléotides constitutifs des ADN (dCTP, dATP, dGTP, dTTP) ou des ARN (dCTP, dATP, dGTP, dUTP) et/ou,
- une ou plusieurs enzymes de restriction, et, le cas échéant, des fragments de restriction de référence caractéristiques des sous-espèces d'*Erwinia carotovora* et/ou d'*Erwinia chrysanthemi,* notamment des fragments de restriction représentés sur la figure 3, dans le cas des sous-espèces d'*Erwinia carotovora* et/ou,
- une ligase et un (ou plusieurs) couple(s) de séquences oligonucléotidiques, susceptibles de s'hybrider de part et d'autre d'un (ou plusieurs) nucléotide(s) situé(s) dans des gènes ou fragment de gènes codant des pectate-lyases, et étant caractéristique(s) d'une espèce, sous-espèce ou pathovar d'*Erwinia,* et/ou
- une (ou plusieurs) sonde(s) oligonucléotidique(s) spécifique(s) d'une région nucléotidique caractéristique d'une espèce, sous-espèce ou pathovar déterminé de bactéries du genre *Erwinia.*

## Patentansprüche (Patentansprüche für folgende(n) Vertragsstaat(en): AT, BE, CH, DE, DK, FR, GB, GR, IE, IT, LI, LU, MC, NL, PT, SE)

1. Ein Verfahren zur Identifizierung und zur Feststellung der Anwesenheit von pektinolytischen Bakterien einer bestimmten Art der Gattung Erwinia, insbesondere der Art *Erwinia chrysanthemi* und der Art *Erwinia carotovora* im Erdreich oder in Wasser oder bei einem Wirt, der dafür geeignet ist, Träger solcher Bakterien zu sein, vorzugsweise bei Pflanzen und Samen, welches Verfahren die folgenden Schritte umfasst:
das Suchen nach DNS- oder RNS-Fragmenten, die für die jeweils betreffende Art, die Unterart oder den Pathovar spezifisch sind und die für eine Pektat-Lyase (PL) codieren, und
das Durchführen einer molekularen Hybridisierung mit mindestens einer spezifischen Sonde, welcher Hybridisierung eine Vervielfältigung der Anzahl der Kopien der oben erwähnten DNS-Fragmente mit Hilfe spezifischer Initiatoren (Startermoleküle) vorausgegangen ist.

2. Eine Nukleotidsequenz, dadurch gekennzeichnet, daß sie die nachfolgende Nukleotidsequenz A ganz oder teilweise enthält: 5'-TTACCGGACGCCGAGCTGTGGCGT-3' oder die dazu komplementäre Sequenz oder eine von der Sequenz A, vorzugsweise durch Unterdrücken und/oder Austausch und/oder Hinzufügen von Nukleotiden abgeleitete Sequenz enthält, und welche Sequenz geeignet ist, sich mit dem Fragment, welches durch die Positionen 1231 und 1254 der in Figur 1 dargestellten Sequenz begrenzt wird, zu hybridisieren, oder die zu dieser abgeleiteten Sequenz komplementäre Sequenz.

3. Eine Nukleotidsequenz, dadurch gekennzeichnet, daß sie die nachfolgende Nukleotidsequenz B ganz oder teilweise enthält, welche ausgewählt ist aus der Gesamtheit oder einem Teil von:
* den nachfolgenden Nukleotidketten: 
5'-CAGGAAGATGTCGTTATCGCGAGT-3'
.........C..............
.........T..............
 oder deren komplementären Sequenzen,
* oder einer von der Sequenz B, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 1642 und 1665 der in Figur 1 dargestellten Sequenz begrenzt wird, zu hybridisieren, oder die zu dieser abgeleiteten Sequenz komplementäre Sequenz.

4. Eine Nukleotidsequenz, dadurch gekennzeichnet, daß sie die nachfolgende Nukleotidsequenz C ganz oder teilweise enthält, welche ausgewählt ist aus der Gesamtheit oder einem Teil von:
* den nachfolgenden Nukleotidketten: 
5'-GATCAGAAAGCCCGCAGCCAGAT-3'
.....A........T........
...........G..T........
 (die Punkte stellen jeweils Nukleotide dar, die mit denen in der ersten Zeile identisch sind) oder deren komplementären Sequenzen,
* oder einer von der Sequenz C, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 295 und 317 der in Figur 2 dargestellten Sequenz begrenzt wird, zu hybridisieren, oder die zu dieser abgeleiteten Sequenz komplementäre Sequenz.

5. Eine Nukleotidsequenz, dadurch gekennzeichnet, daß sie die nachfolgende Nukleotidsequenz D ganz oder teilweise enthält, welche ausgewählt ist aus der Gesamtheit oder einem Teil von:
* den nachfolgenden Nukleotidketten: 
5'-CTGTGGCCGATCAGGATGGTTTTGTCGTGC-3'
..A.......................A...
T.............C...............
.....A..A.....C...............
.................A............
 oder deren komplementären Sequenzen,
* oder einer von der Sequenz D, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 672 und 701 der in Figur 2 dargestellten Sequenz begrenzt wird, zu hybridisieren, oder die zu dieser abgeleiteten Sequenz komplementäre Sequenz.

6. Initiatorpaare (Startermolekülpaare) für die genetische Vervielfältigung, dadurch gekennzeichnet, daß der eine der Initiatoren dieser Paare aus den Sequenzen ausgewählt wird, welche die Nukleotidsequenz A oder eine davon abgeleitete Sequenz gemäß Anspruch 2 ganz oder teilweise enthalten, während dagegen der andere Initiator aus den Sequenzen ausgewählt wird, welche die Nukleotidsequenz B oder eine davon abgeleitete Sequenz gemäß Anspruch 3 ganz oder teilweise enthalten.

7. Initiatorpaare für die genetische Vervielfältigung, dadurch gekennzeichnet, daß der eine der Initiatoren dieser Paare aus den Sequenzen ausgewählt wird, welche die Nukleotidsequenz C oder eine davon abgeleitete Sequenz gemäß Anspruch 4 ganz oder teilweise enthalten, während dagegen der andere Initiator aus den Sequenzen ausgewählt wird, welche die Nukleotidsequenz D oder eine davon abgeleitete Sequenz gemäß Anspruch 5 ganz oder teilweise enthalten.

8. Nukleotidische Sonde, dadurch gekennzeichnet, daß sie mindestens 10 Nukleotide aus der Sequenz enthält, welche durch die Nukleotide begrenzt wird, die sich an den Positionen 1231 und 1665 der in Figur 1 dargestellten Sequenz befinden, oder mindestens 10 Nukleotide enthält, welche eine Übereinstimmung von mindestens 40 % mit der oben erwähnten Sequenz aufweisen.

9. Nukleotidische Sonde, dadurch gekennzeichnet, daß sie mindestens 10 Nukleotide aus der Sequenz enthält, welche durch die Nukleotide begrenzt wird, die sich an den Positionen 295 und 701 der in Figur 2 dargestellten Sequenz befinden, oder mindestens 10 Nukleotide enthält, welche eine Übereinstimmung von mindestens 40 % mit der oben erwähnten Sequenz aufweisen.

10. Nukleotidsequenzen gemäß einem der Ansprüche 2 bis 9, dadurch gekennzeichnet, daß sie markiert sind, vorzugsweise im Wege einer radioaktiven oder enzymatischen Methode, mittels einer fluoreszierenden Zusammensetzung, durch Bindung an ein antigenes Molekül (das zur Erkennung durch die Antikörper geeignet ist), oder an ein ganz anderes Molekül, das geeignet ist, direkt oder indirekt mit Hilfe von Reagenzien nachgewiesen zu werden, wobei diese besagte Bindung durch die Vermittlung eines Bindungarmes bewirkt werden kann, vorzugsweise durch die Vermittlung einer Nukleotidseqzuenz, die aus ungefähr 5 bis 100 Nukleotiden besteht und die an den äußersten Positionen 3' oder 5' dieser Sequenzen angeordnet ist.

11. Ein Verfahren gemäß Anspruch 1 zur Identifizierung und zur Feststellung der Anwesenheit von *Erwinia carotovora* im Erdreich oder in Wasser oder bei einem Wirt, vorzugsweise bei Pflanzen und Samen, welches Verfahren die folgenden Stufen umfasst:
- die Behandlung einer aus dem Erdreich, aus dem Wasser oder dem besagten Wirt entnommenen Probe in einer Weise, daß das Genom von *Erwinia carotovora* für die im Anspruch 6 definierten Initiatoren und, im Bedarfsfall, für jede DNS- oder RNS-Polymerase zugänglich wird, was eine Replikation der beiden Stränge der genomen DNS oder entsprechend analog der RNS erlaubt, und welche Behandlung vorzugsweise durch Erhitzen, Maceration, (in einem Lösungsmittel wie z.B. Wasser), Zermahlen oder Ultraschalleinwirkung durchgeführt wird,
- die Vervielfältigung der Anzahl der Kopien der Fragmente der für die PL kodierenden Gene, die möglicherweise in dieser Probe anwesend sind, mit Hilfe der Initiatorpaare, die vorstehend definiert sind,
- das Feststellen der möglichen Anwesenheit der für die PL kodierenden Gene und somit der Anwesenheit von *Erwinia carotovora* in der untersuchten Probe mit Hilfe einer Sonde gemäß Anspruch 8, welche im Bedarfsfall markiert ist, und zwar vorzugsweise in der im Anspruch 10 angezeigten Weise.

12. Ein Verfahren gemäß Anspruch 1 zur Identifizierung und zur Feststellung der Anwesenheit von *Erwinia chrysanthemi* im Erdreich oder in Wasser oder bei einem Wirt, vorzugsweise bei Pflanzen und Samen, welches Verfahren die folgenden Stufen umfasst:
- die Behandlung einer aus dem Erdreich, aus dem Wasser oder dem besagten Wirt entnommenen Probe in einer Weise, daß das Genom von *Erwinia chrysanthemi* für die im Anspruch 7 definierten Initiatoren und, im Bedarfsfall für jede DNS- oder RNS-Polymerase zugänglich wird, was eine Replikation der beiden Stränge der genomen DNS oder entsprechend analog der RNS erlaubt, und welche Behandlung vorzugsweise durch Erhitzen, Maceration, (in einem Lösungsmittel wie z.B. Wasser), Zermahlen oder Ultraschalleinwirkung durchgeführt wird,
- die Vervielfältigung der Anzahl der Kopien der Fragmente der für die PL kodierenden Gene, die möglicherweise in dieser Probe anwesend sind, mit Hilfe der Initiatorpaare, die vorstehend definiert sind,
- die Feststellung der möglichen Anwesenheit der für die PL kodierenden Gene und somit der Anwesenheit von *Erwinia chrysanthemi* in der untersuchten Probe mit Hilfe einer Sonde gemäß Anspruch 9, welche im Bedarfsfall markiert ist, und zwar vorzugsweise in der im Anspruch 10 angezeigten Weise.

13. Ein Verfahren gemäß Anspruch 11 oder 12, dadurch gekennzeichnet, daß der Schritt der Vervielfältigung der Anzahl der für die Pektat-Lyasen kodierenden Gene die folgenden Stufen umfasst:
- eine Vorstufe der Denaturierung des DNS-Doppelstranges zum DNS-Einzelstrang, durch Erwärmen im Temperaturbereich zwischen etwa 80 °C und etwa 110 °C, vorzugsweise bei 100 °C, vorzugsweise in einem Puffer, der aus Tris-HCl (10 mM pH 8,3), 50 mM KCl, 1,5 mM MgCl₂, 0,01 % Gelatine, DNS-(oder RNS)-Polymerase-Kofaktoren, insbesondere Mg²⁺-Ionen und K⁺-Ionen, den 4-Desoxynukleotiden, aus welchen sich die DNS (dCTP, dATP, dGTP, dTTP) oder die RNS (dCTP, dUTP, dGTP, dTTP) zusammensetzt, und den in den Ansprüchen 6 und 7 definierten Initiatorpaaren besteht,
- die eigentliche Vervielfältigung durch Zugabe von beispielsweise thermoresistenter DNS-Polymerase zum in der vorstehenden Stufe erhaltenen Medium, und
- das Erwärmen auf ca. 94 °C, was der Stufe der eigentlichen Denaturierung entspricht,
- dann das Erwärmen auf den Bereich von ca. 60 °C bis ca. 76 °C, was der Stufe der Hybridisierung der Initiatorpaare mit den für die PL kodierenden Genen oder den Fragmenten dieser Gene entspricht, welche möglicherweise in der untersuchten, biologischen Probe anwesend sind,
- und schließlich das Erwärmen auf den Bereich von ca. 50 °C bis ca. 76 °C, was der Stufe der Verlängerung der in der vorhergehenden Stufe gegenseitig hybridisierten Initiatorpaare entspricht, um somit Nukleotidsequenzen herzustellen, die gänzlich oder teilweise komplementär zu den genomen Sequenzen der pektinolytischen *Erwinia*-Bakterien sind, wobei die letztgenannten Sequenzen durch die Nukleotide begrenzt werden, die sich mit den oben erwähnten Initiatoren hybridisierten,
- die etwa 20- bis etwa 50-fache, vorzugsweise 25-bis 35-fache Wiederholung der vorhergehenden Vervielfältigungsstufe;
- im Bedarfsfall die Wiederverwendung eines aliquoten Anteils des am Ende der vorhergehenden Vervielfältigungsstufe erhaltenen Mediums, um eine neue Vervielfältigung gemäß der vorstehend beschriebenen Methode zu realisieren.

14. Anwendung eines Verfahrens gemäß einem der Ansprüche 11 bis 13 zur Durchführung einer Diagnosemethode für das mögliche Auftreten von Krankheiten, die durch die Bakterien der Art *Erwinia carotovora* und insbesondere durch eine der Unterarten *Ecc, Eca, Eco* und *Ecw,* durch die Bakterien der Art *Erwinia chrysanthemi,* oder durch jegliche Art, Unterart oder Pathovar verursacht werden, und zwar sowohl durch die tatsächlich bekannten, als auch durch die später durch Modifikation der bekannten Taxonomie davon abgeleiteten.

15. Ausstattung zur Durchführung eines Verfahrens gemäß einem der Ansprüche 11 bis 13, dadurch gekennzeichnet, daß sie folgendes umfasst:
- mindestens ein Initiatorpaar gemäß Anspruch 6, und/oder
- mindestens ein Initiatorpaar gemäß Anspruch 7,
- eine (oder mehrere) Sonde(n) gemäß Anspruch 8,
und/oder eine (oder mehrere) Sonde(n) gemäß Anspruch 9, welche besagten Sonden geeignet sind, sich gänzlich oder teilweise mit den Fragmenten der mit Hilfe der Initiatorpaare gemäß den Ansprüchen 6 oder 7 vervielfältigten Gene zu hybridisieren.

16. Ausstattung gemäß Anspruch 15, dadurch gekennzeichnet, daß sie außerdem folgendes umfasst:
- eine thermoresistente DNS- oder RNS-Polymerase,
- ein Reaktionsmedium, das vorzugsweise aus Tris-HCl (10 mM pH 8,3), 50 mM KCl, 1,5 mM MgCl₂, 0,01 % Gelatine, DNS-Polymerase-Kofaktoren, vorzugsweise Mg²⁺-Ionen und K⁺-Ionen, den 4-Desoxynukleotiden, aus welchen sich die DNS (dCTP, dATP, dGTP, dTTP) oder die RNS (dCTP, dUTP, dGTP, dTTP) zusammensetzt, besteht, und/oder,
- ein oder mehrere Restriktionsenzym(e), und, im Bedarfsfall, Restriktionsfragmente, die einen charakteristischen Bezug zu den Unterarten der *Erwinia carotovora* und/oder der *Erwinia chrysanthema* aufweisen, im Fall der Unterarten der *Erwinia carotovora* vorzugsweise die in Figur 3 dargestellten Restriktionsfragmente, und/oder
- eine Ligase und ein (oder mehrere) Oligonukleotidsequenzenpaare, welche(s) geeignet sind/(ist), sich wechselseitig mit einem (oder mehreren) Nukleotid(en) zu hybridisieren, welche(s) in den für Pektat-Lyasen kodierenden Genen oder Fragmenten dieser Gene angeordnet ist/(sind), und welche(s) Oligonukleotidsequenzpaar(e) charakteristisch für eine Art oder Unterart oder Pathovar von Bakterien der Gattung *Erwinia* ist/(sind), und/oder,
- eine (oder mehrere) Oligonukleotidsonde(n), welche für einen Nukleotidabschnitt spezifisch ist/(sind), der charakteristisch für eine Art oder Unterart oder Pathovar von Bakterien der Gattung *Erwinia* ist/(sind).

17. Verwendung von Nukleotidsequenzen, die gänzlich oder teilweise den Genen entsprechen, die im Inneren des Genoms pektinolytischer Bakterien der Gattung *Erwinia,* und insbesondere von *Erwinia carotovora* und von *Erwinia chrysanthemi,* für Pektat-Lyasen kodieren, zur Durchführung eines Verfahrens zur Identifizierung von Unterarten, und/oder von Stämmen oder von Pathovaren der Arten oder der Unterarten verschiedener Bakterien der Gattung *Erwinia.*

## Patentansprüche (Patentansprüche für folgende(n) Vertragsstaat(en): ES)

1. Ein Verfahren zur Identifizierung und zur Feststellung der Anwesenheit von pektinolytischen Bakterien einer bestimmten Art der Gattung *Erwinia*, insbesondere der Art *Erwinia chrysanthemi* und der Art *Erwinia carotovora* im Erdreich oder in Wasser oder bei einem Wirt, der dafür geeignet ist, Träger solcher Bakterien zu sein, vorzugsweise bei Pflanzen und Samen, welches Verfahren die folgenden Schritte umfasst:
das Suchen nach DNS- oder RNS-Fragmenten, die für die jeweils betreffende Art, die Unterart oder den Pathovar spezifisch sind und die für eine Pektat-Lyase (PL) codieren, und
das Durchführen einer molekularen Hybridisierung mit mindestens einer spezifischen Sonde, welcher Hybridisierung eine Vervielfältigung der Anzahl der Kopien der oben erwähnten DNS-Fragmente mit Hilfe spezifischer Initiatoren (Startermoleküle) vorausgegangen ist.

2. Eine Nukleotidsequenz, dadurch gekennzeichnet, daß sie die nachfolgende Nukleotidsequenz A ganz oder teilweise enthält: 5'-TTACCGGACGCCGAGCTGTGGCGT-3' oder die dazu komplementäre Sequenz oder eine von der Sequenz A, vorzugsweise durch Unterdrücken und/oder Austausch und/oder Hinzufügen von Nukleotiden abgeleitete Sequenz enthält, und welche Sequenz geeignet ist, sich mit dem Fragment, welches durch die Positionen 1231 und 1254 der in Figur 1 dargestellten Sequenz begrenzt wird, zu hybridisieren, oder die zu dieser abgeleiteten Sequenz komplementäre Sequenz.

3. Eine Nukleotidsequenz, dadurch gekennzeichnet, daß sie die nachfolgende Nukleotidsequenz B ganz oder teilweise enthält, welche ausgewählt ist aus der Gesamtheit oder einem Teil von:
* den nachfolgenden Nukleotidketten: 
5'-CAGGAAGATGTCGTTATCGCGAGT-3'
.........C..............
.........T..............
 oder deren komplementären Sequenzen,
* oder einer von der Sequenz B, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 1642 und 1665 der in Figur 1 dargestellten Sequenz begrenzt wird, zu hybridisieren, oder die zu dieser abgeleiteten Sequenz komplementäre Sequenz.

4. Eine Nukleotidsequenz, dadurch gekennzeichnet, daß sie die nachfolgende Nukleotidsequenz C ganz oder teilweise enthält, welche ausgewählt ist aus der Gesamtheit oder einem Teil von:
* den nachfolgenden Nukleotidketten: 
5'-GATCAGAAAGCCCGCAGCCAGAT-3'
.....A.........T.......
............G..T.......
 (die Punkte stellen jeweils Nukleotide dar, die mit denen in der ersten Zeile identisch sind) oder deren komplementären Sequenzen,
* oder einer von der Sequenz C, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 295 und 317 der in Figur 2 dargestellten Sequenz begrenzt wird, zu hybridisieren, oder die zu dieser abgeleiteten Sequenz komplementäre Sequenz.

5. Eine Nukleotidsequenz, dadurch gekennzeichnet, daß sie die nachfolgende Nukleotidsequenz D ganz oder teilweise enthält, welche ausgewählt ist aus der Gesamtheit oder einem Teil von:
* den nachfolgenden Nukleotidketten: 
5'-CTGTGGCCGATCAGGATGGTTTTGTCGTGC-3'
..A.......................A...
T.............C...............
.....A..A.....C...............
.................A............
 oder deren komplementären Sequenzen,
* oder einer von der Sequenz D, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 672 und 701 der in Figur 2 dargestellten Sequenz begrenzt wird, zu hybridisieren, oder die zu dieser abgeleiteten Sequenz komplementäre Sequenz.

6. Initiatorpaare (Startermolekülpaare) für die genetische Vervielfältigung, dadurch gekennzeichnet, daß der eine der Initiatoren dieser Paare aus den Sequenzen ausgewählt wird, welche die Nukleotidsequenz A oder eine davon abgeleitete Sequenz gemäß Anspruch 2 ganz oder teilweise enthalten, während dagegen der andere Initiator aus den Sequenzen ausgewählt wird, welche die Nukleotidsequenz B oder eine davon abgeleitete Sequenz gemäß Anspruch 3 ganz oder teilweise enthalten.

7. Initiatorpaare für die genetische Vervielfältigung, dadurch gekennzeichnet, daß der eine der Initiatoren dieser Paare aus den Sequenzen ausgewählt wird, welche die Nukleotidsequenz C oder eine davon abgeleitete Sequenz gemäß Anspruch 4 ganz oder teilweise enthalten, während dagegen der andere Initiator aus den Sequenzen ausgewählt wird, welche die Nukleotidsequenz D oder eine davon abgeleitete Sequenz gemäß Anspruch 5 ganz oder teilweise enthalten.

8. Nukleotidische Sonde, dadurch gekennzeichnet, daß sie mindestens 10 Nukleotide aus der Sequenz enthält, welche durch die Nukleotide begrenzt wird, die sich an den Positionen 1231 und 1665 der in Figur 1 dargestellten Sequenz befinden, oder mindestens 10 Nukleotide enthält, welche eine Übereinstimmung von mindestens 40 % mit der oben erwähnten Sequenz aufweisen.

9. Nukleotidische Sonde, dadurch gekennzeichnet, daß sie mindestens 10 Nukleotide aus der Sequenz enthält, welche durch die Nukleotide begrenzt wird, die sich an den Positionen 295 und 701 der in Figur 2 dargestellten Sequenz befinden, oder mindestens 10 Nukleotide enthält, welche eine Übereinstimmung von mindestens 40 % mit der oben erwähnten Sequenz aufweisen.

10. Nukleotidsequenzen gemäß einem der Ansprüche 2 bis 9, dadurch gekennzeichnet, daß sie markiert sind, vorzugsweise im Wege einer radioaktiven oder enzymatischen Methode, mittels einer fluoreszierenden Zusammensetzung, durch Bindung an ein antigenes Molekül (das zur Erkennung durch die Antikörper geeignet ist), oder an ein ganz anderes Molekül, das geeignet ist, direkt oder indirekt mit Hilfe von Reagenzien nachgewiesen zu werden, wobei diese besagte Bindung durch die Vermittlung eines Bindungarmes bewirkt werden kann, vorzugsweise durch die Vermittlung einer Nukleotidseqzuenz, die aus ungefähr 5 bis 100 Nukleotiden besteht und die an den äußersten Positionen 3' oder 5' dieser Sequenzen angeordnet ist.

11. Ein Verfahren gemäß Anspruch 1 zur Identifizierung und zur Feststellung der Anwesenheit von *Erwinia carotovora* im Erdreich oder in Wasser oder bei einem Wirt, vorzugsweise bei Pflanzen und Samen, welches Verfahren die folgenden Stufen umfasst:
- die Behandlung einer aus dem Erdreich, aus dem Wasser oder dem besagten Wirt entnommenen Probe in einer Weise, daß das Genom von *Erwinia carotovora* für die im Anspruch 6 definierten Initiatoren und, im Bedarfsfall, für jede DNS- oder RNS-Polymerase zugänglich wird, was eine Replikation der beiden Stränge der genomen DNS oder entsprechend analog der RNS erlaubt, und welche Behandlung vorzugsweise durch Erhitzen, Maceration, (in einem Lösungsmittel wie z.B. Wasser), Zermahlen oder Ultraschalleinwirkung durchgeführt wird,
- die Vervielfältigung der Anzahl der Kopien der Fragmente der für die PL kodierenden Gene, die möglicherweise in dieser Probe anwesend sind, mit Hilfe der Initiatorpaare, die vorstehend definiert sind,
- das Feststellen der möglichen Anwesenheit der für die PL kodierenden Gene und somit der Anwesenheit von *Erwinia carotovora* in der untersuchten Probe mit Hilfe einer Sonde gemäß Anspruch 8, welche im Bedarfsfall markiert ist, und zwar vorzugsweise in der im Anspruch 10 angezeigten Weise.

12. Ein Verfahren gemäß Anspruch 1 zur Identifizierung und zur Feststellung der Anwesenheit von *Erwinia chrysanthemi* im Erdreich oder in Wasser oder bei einem Wirt, vorzugsweise bei Pflanzen und Samen, welches Verfahren die folgenden Stufen umfasst:
- die Behandlung einer aus dem Erdreich, aus dem Wasser oder dem besagten Wirt entnommenen Probe in einer Weise, daß das Genom von *Erwinia chrysanthemi* für die im Anspruch 7 definierten Initiatoren und, im Bedarfsfall für jede DNS- oder RNS-Polymerase zugänglich wird, was eine Replikation der beiden Stränge der genomen DNS oder entsprechend analog der RNS erlaubt, und welche Behandlung vorzugsweise durch Erhitzen, Maceration, (in einem Lösungsmittel wie z.B. Wasser), Zermahlen oder Ultraschalleinwirkung durchgeführt wird,
- die Vervielfältigung der Anzahl der Kopien der Fragmente der für die PL kodierenden Gene, die möglicherweise in dieser Probe anwesend sind, mit Hilfe der Initiatorpaare, die vorstehend definiert sind,
- die Feststellung der möglichen Anwesenheit der für die PL kodierenden Gene und somit der Anwesenheit von *Erwinia chrysanthemi* in der untersuchten Probe mit Hilfe einer Sonde gemäß Anspruch 9, welche im Bedarfsfall markiert ist, und zwar vorzugsweise in der im Anspruch 10 angezeigten Weise.

13. Ein Verfahren gemäß Anspruch 11 oder 12, dadurch gekennzeichnet, daß der Schritt der Vervielfältigung der Anzahl der für die Pektat-Lyasen kodierenden Gene die folgenden Stufen umfasst:
- eine Vorstufe der Denaturierung des DNS-Doppelstranges zum DNS-Einzelstrang, durch Erwärmen im Temperaturbereich zwischen etwa 80 °C und etwa 110 °C, vorzugsweise bei 100 °C, vorzugsweise in einem Puffer, der aus Tris-HCl (10 mM pH 8,3), 50 mM KCl, 1,5 mM MgCl₂, 0,01 % Gelatine, DNS-(oder RNS)-Polymerase-Kofaktoren, insbesondere Mg²⁺-Ionen und K⁺-Ionen, den 4-Desoxynukleotiden, aus welchen sich die DNS (dCTP, dATP, dGTP, dTTP) oder die RNS (dCTP, dUTP, dGTP, dTTP) zusammensetzt, und den in den Ansprüchen 6 und 7 definierten Initiatorpaaren besteht,
- die eigentliche Vervielfältigung durch Zugabe von beispielsweise thermoresistenter DNS-Polymerase zum in der vorstehenden Stufe erhaltenen Medium, und
- das Erwärmen auf ca. 94 °C, was der Stufe der eigentlichen Denaturierung entspricht,
- dann das Erwärmen auf den Bereich von ca. 60 °C bis ca. 76 °C, was der Stufe der Hybridisierung der Initiatorpaare mit den für die PL kodierenden Genen oder den Fragmenten dieser Gene entspricht, welche möglicherweise in der untersuchten, biologischen Probe anwesend sind,
- und schließlich das Erwärmen auf den Bereich von ca. 50 °C bis ca. 76 °C, was der Stufe der Verlängerung der in der vorhergehenden Stufe gegenseitig hybridisierten Initiatorpaare entspricht, um somit Nukleotidsequenzen herzustellen, die gänzlich oder teilweise komplementär zu den genomen Sequenzen der pektinolytischen *Erwinia*-Bakterien sind, wobei die letztgenannten Sequenzen durch die Nukleotide begrenzt werden, die sich mit den oben erwähnten Initiatoren hybridisierten,
- die etwa 20- bis etwa 50-fache, vorzugsweise 25-bis 35-fache Wiederholung der vorhergehenden Vervielfältigungsstufe;
- im Bedarfsfall die Wiederverwendung eines aliquoten Anteils des am Ende der vorhergehenden Vervielfältigungsstufe erhaltenen Mediums, um eine neue Vervielfältigung gemäß der vorstehend beschriebenen Methode zu realisieren.

14. Anwendung eines Verfahrens gemäß einem der Ansprüche 11 bis 13 zur Durchführung einer Diagnosemethode für das mögliche Auftreten von Krankheiten, die durch die Bakterien der Art *Erwinia carotovora* und insbesondere durch eine der Unterarten *Ecc*, *Eca*, *Eco* und *Ecw*, durch die Bakterien der Art *Erwinia chrysanthemi,* oder durch jegliche Art, Unterart oder Pathovar verursacht werden, und zwar sowohl durch die tatsächlich bekannten, als auch durch die später durch Modifikation der bekannten Taxonomie davon abgeleiteten.

15. Ausstattung zur Durchführung eines Verfahrens gemäß einem der Ansprüche 11 bis 13, dadurch gekennzeichnet, daß sie folgendes umfasst:
- mindestens ein Initiatorpaar gemäß Anspruch 6, und/oder
- mindestens ein Initiatorpaar gemäß Anspruch 7,
- eine (oder mehrere) Sonde(n) gemäß Anspruch 8,
und/oder eine (oder mehrere) Sonde(n) gemäß Anspruch 9, welche besagten Sonden geeignet sind, sich gänzlich oder teilweise mit den Fragmenten der mit Hilfe der Initiatorpaare gemäß den Ansprüchen 6 oder 7 vervielfältigten Gene zu hybridisieren.

16. Ausstattung gemäß Anspruch 15, dadurch gekennzeichnet, daß sie außerdem folgendes umfasst:
- eine thermoresistente DNS- oder RNS-Polymerase,
- ein Reaktionsmedium, das vorzugsweise aus Tris-HCl (10 mM pH 8,3), 50 mM KCl, 1,5 mM MgCl₂, 0,01 % Gelatine, DNS-Polymerase-Kofaktoren, vorzugsweise Mg²⁺-Ionen und K⁺-Ionen, den 4-Desoxynukleotiden, aus welchen sich die DNS (dCTP, dATP, dGTP, dTTP) oder die RNS (dCTP, dUTP, dGTP, dTTP) zusammensetzt, besteht, und/oder,
- ein oder mehrere Restriktionsenzym(e), und, im Bedarfsfall, Restriktionsfragmente, die einen charakteristischen Bezug zu den Unterarten der *Erwinia carotovora* und/oder der *Erwinia chrysanthema* aufweisen, im Fall der Unterarten der *Erwinia carotovora* vorzugsweise die in Figur 3 dargestellten Restriktionsfragmente, und/oder
- eine Ligase und ein (oder mehrere) Oligonukleotidsequenzenpaare, welche(s) geeignet sind/(ist), sich wechselseitig mit einem (oder mehreren) Nukleotid(en) zu hybridisieren, welche(s) in den für Pektat-Lyasen kodierenden Genen oder Fragmenten dieser Gene angeordnet ist/(sind), und welche(s) Oligonukleotidsequenzpaar(e) charakteristisch für eine Art oder Unterart oder Pathovar von Bakterien der Gattung Erwinia ist/(sind), und/oder,
- eine (oder mehrere) Oligonukleotidsonde(n), welche für einen Nukleotidabschnitt spezifisch ist/(sind), der charakteristisch für eine Art oder Unterart oder Pathovar von Bakterien der Gattung *Erwinia* ist/(sind).

17. Verwendung von Nukleotidsequenzen, die gänzlich oder teilweise den Genen entsprechen, die im Inneren des Genoms pektinolytischer Bakterien der Gattung *Erwinia*, und insbesondere von *Erwinia carotovora* und von *Erwinia chrysanthemi,* für Pektat-Lyasen kodieren, zur Durchführung eines Verfahrens zur Identifizierung von Unterarten, und/oder von Stämmen oder von Pathovaren der Arten oder der Unterarten verschiedener Bakterien der Gattung *Erwinia.*

18. Ein Verfahren zur Herstellung einer Nukleotidsequenz, welche ganz oder teilweise die folgende Nukleotidsequenz A enthält: 5'-TTACCGGACGCCGAGCTGTGGCGT-3' oder der dazu komplementären Sequenz oder einer von der Sequenz A, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 1231 und 1254 der in Figur 1 dargestellten Sequenz begrenzt wird, zu hybridisieren, oder der zu dieser abgeleiteten Sequenz komplementären Sequenz, welches besagte Verfahren entweder auf biologischem Wege durchgeführt wird, vorzugsweise nach Transformation und Klonierung der zellulären Wirte, die geeignet erscheinen, um diese Nukleotidsequenz zu enthalten, oder durch Vervielfältigung der Anzahl der Kopien dieser Nukleotidsequenz, vorzugsweise gemäß der PCR-Technik, und zwar entweder auf chemischem Wege, vorzugsweise mittels der automatisierten -Cyanethyl-Phosphoramidit-Methode, die in *Bioorganic Chemistry* 4; Seiten 274-325, 1986 beschrieben ist, oder mittels der Aufbaumethode, zum Beispiel durch Heranführen (oder Verknüpfung mit Hilfe einer Ligase) von auf chemischem Wege synthetisierten Oligonukleotiden, an deren äußersten Positionen angemessene Restriktionsstellen vorgesehen sind, gemäß dem *in Proc. Nat. Acad. Sci*. *USA*, 80; Seiten 7461-7465, 1983 beschriebenen Prinzip; wobei diesen Methoden die Klonierung der somit in einem geeigneten, vorzugsweise plasmidischen Vektor erhaltenen DNS und die Wiedergewinnung der DNS, entweder mittels einer geeigneten Sonde, oder durch Elektrophorese, im allgemeinen nach einer Restriktion und gefolgt von einer Elution der Fragmente folgt.

19. Ein Verfahren zur Herstellung einer Nukleotidsequenz, welche die nachfolgende Nukleotidsequenz B ganz oder teilweise enthält, welche ausgewählt ist aus der Gesamtheit oder einem Teil von:
* den nachfolgenden Nukleotidketten: 
5'-CAGGAAGATGTCGTTATCGCGAGT-3'
.........C..............
.........T..............
 oder deren komplementären Sequenzen,
* oder einer von der Sequenz B, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 1642 und 1665 der in Figur 1 dargestellten Sequenz begrenzt wird, zu hybridisieren, oder der zu dieser abgeleiteten Sequenz komplementären Sequenz, welches Verfahren gemäß den in Anspruch 18 beschriebenen Methoden durchgeführt wird.

20. Ein Verfahren zur Herstellung einer Nukleotidsequenz, welche die nachfolgende Nukleotidsequenz C ganz oder teilweise enthält, welche ausgewählt ist aus der Gesamtheit oder einem Teil von:
* den nachfolgenden Nukleotidketten: 
5'-GATCAGAAAGCCCGCAGCCAGAT-3'
.....A.......T.........
..........G..T.........
 (die Punkte stellen jeweils Nukleotide dar, die mit denen in der ersten Zeile identisch sind) oder deren komplementären Sequenzen,
* oder einer von der Sequenz C, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 295 und 317 der in Figur 2 dargestellten Sequenz begrenzt wird, zu hybridisieren, oder der zu dieser abgeleiteten Sequenz komplementären Sequenz, welches Verfahren gemäß den in Anspruch 18 beschriebenen Methoden durchgeführt wird.

21. Ein Verfahren zur Herstellung einer Nukleotidsequenz, welche die nachfolgende Nukleotidsequenz D ganz oder teilweise enthält, welche ausgewählt ist aus der Gesamtheit oder einem Teil von:
* den nachfolgenden Nukleotidketten: 
5'-CTGTGGCCGATCAGGATGGTTTTGTCGTGC-3'
..A.......................A...
T.............C...............
.....A..A.....C...............
.................A............
 oder deren komplementären Sequenzen,
* oder einer von der Sequenz D, vorzugsweise durch Unterdrücken und/oder Austausch und/oder Hinzufügen von Nukleotiden abgeleiteten Sequenz, und welche Sequenz geeignet ist, sich mit dem Fragment, welches durch die Positionen 672 und 701 der in Figur 2 dargestellten Sequenz begrenzt wird, zu hybridisieren, oder der zu dieser abgeleiteten Sequenz komplementären Sequenz, welches Verfahren gemäß den in Anspruch 18 beschriebenen Methoden durchgeführt wird.

22. Ein Verfahren zur Herstellung von Initiatorpaaren für die genetische Vervielfältigung, in welchem Verfahren der eine der Initiatoren dieses Paares aus den Sequenzen ausgewählt wird, welche die Nukleotidsequenz A oder gemäß Anspruch 18 eine davon abgeleitete Sequenz ganz oder teilweise enthalten, während dagegen der andere Initiator aus den Sequenzen ausgewählt wird, welche die Nukleotidsequenz B gemäß Anspruch 19 oder eine davon abgeleitete Sequenz ganz oder teilweise enthalten, und wobei das besagte Verfahren gemäß den in Anspruch 18 beschriebenen Methoden durchgeführt wird.

23. Ein Verfahren zur Herstellung von Initiatorpaaren für die genetische Vervielfältigung, in welchem Verfahren der eine der Initiatoren dieses Paares aus den Sequenzen ausgewählt wird, welche die Nukleotidsequenz C gemäß Anspruch 20 oder eine davon abgeleitete Sequenz ganz oder teilweise enthalten, während dagegen der andere Initiator aus den Sequenzen ausgewählt wird, welche die Nukleotidsequenz D gemäß Anspruch 21 oder eine davon abgeleitete Sequenz ganz oder teilweise enthalten, und wobei das besagte Verfahren gemäß den in Anspruch 18 beschriebenen Methoden durchgeführt wird.

24. Verfahren zur Herstellung einer nukleotidischen Sonde, welche mindestens 10 Nukleotide aus der Sequenz enthält, welche durch die Nukleotide begrenzt wird, die sich an den Positionen 1231 und 1665 der in Figur 1 dargestellten Sequenz befinden, oder mindestens 10 Nukleotide enthält, welche eine Übereinstimmung von mindestens 40 % mit der oben erwähnten Sequenz aufweisen, und welches Verfahren gemäß den in Anspruch 18 beschriebenen Methoden durchgeführt wird.

25. Verfahren zur Herstellung einer nukleotidischen Sonde, welche mindestens 10 Nukleotide aus der Sequenz enthält, welche durch die Nukleotide begrenzt wird, die sich an den Positionen 295 und 701 der in Figur 2 dargestellten Sequenz befinden, oder mindestens 10 Nukleotide enthält, welche eine Übereinstimmung von mindestens 40 % mit der oben erwähnten Sequenz aufweisen, und welches Verfahren gemäß den in Anspruch 18 beschriebenen Methoden durchgeführt wird.

26. Verfahren zur Herstellung von Nukleotidsequenzen, so wie sie in einem der Ansprüche 18 bis 25 definiert sind, welche Sequenzen markiert sind, und zwar vorzugsweise im Wege einer radioaktiven oder enzymatischen Methode, mittels einer fluoreszierenden Zusammensetzung, durch Bindung an ein antigenes Molekül (das zur Erkennung durch die Antikörper geeignet ist), oder an ein ganz anderes Molekül, das geeignet ist, direkt oder indirekt mit Hilfe von Reagenzien nachgewiesen zu werden, wobei diese besagte Bindung durch die Vermittlung eines Bindungarmes bewirkt werden kann, vorzugsweise durch die Vermittlung einer Nukleotidseqzuenz, die aus ungefähr 5 bis 100 Nukleotiden besteht und die an den äußersten Positionen 3' oder 5' dieser Sequenzen angeordnet ist, und welches Verfahren gemäß den in Anspruch 18 beschriebenen Methoden durchgeführt wird.

27. Verfahren zur Herstellung einer Ausstattung für die Durchführung eines Verfahrens gemäß einem der Ansprüche 11 bis 13, welches besagte Verfahren eine Stufe zum Zusammenstellen des folgenden umfasst:
- mindestens ein Initiatorpaar, so wie in Anspruch 22 definiert, und/oder
- mindestens ein Initiatorpaar, so wie in Anspruch 23 definiert,
- eine (oder mehrere) Sonde(n), so wie in Anspruch 24 definiert, und/oder eine (oder mehrere) Sonde(n), die im Anspruch 25 definiert sind, welche besagten Sonden geeignet sind, sich gänzlich oder teilweise mit den Fragmenten der mit Hilfe der Initiatorpaare, so wie in den Ansprüchen 22 und 23 entsprechend definiert, vervielfältigten Gene zu hybridisieren.

28. Verfahren zur Herstellung einer Ausstattung gemäß Anspruch 27, welches besagte Verfahren außerdem eine Stufe zum Zusammenstellen des folgenden umfasst:
- eine thermoresistente DNS- oder RNS-Polymerase,
- ein Reaktionsmedium, das vorzugsweise aus Tris-HCl (10 mM pH 8,3), 50 mM KCl, 1,5 mM MgCl₂, 0,01 % Gelatine, DNS-Polymerase-Kofaktoren, vorzugsweise Mg²⁺-Ionen und K⁺-Ionen, den 4-Desoxynukleotiden, aus welchen sich die DNS (dCTP, dATP, dGTP, dTTP) oder die RNS (dCTP, dUTP, dGTP, dTTP) zusammensetzt, besteht, und/oder,
- ein oder mehrere Restriktionsenzym(e), und, im Bedarfsfall, die Restriktionsfragmente, die einen charakteristischen Bezug zu den Unterarten der *Erwinia carotovora* und/oder der *Erwinia chrysanthema* aufweisen, im Fall der Unterarten der *Erwinia carotovora* vorzugsweise die in Figur 3 dargestellten Restriktionsfragmente, und/oder
- eine Ligase und ein (oder mehrere) Oligonukleotidsequenzenpaare, welche(s) geeignet sind/(ist), sich wechselseitig mit einem (oder mehreren) Nukleotid(en) zu hybridisieren, welche(s) in den für Pektat-Lyasen kodierenden Genen oder Fragmenten dieser Gene angeordnet ist/(sind), und welche(s) Oligonukleotidsequenzpaar(e) charakteristisch für eine Art oder Unterart oder Pathovar von Bakterien der Gattung *Erwinia* ist/(sind), und/oder,
- eine (oder mehrere) Oligonukleotidsonde(n), welche für einen Nukleotidabschnitt spezifisch ist/(sind), der charakteristisch für eine Art oder Unterart oder Pathovar von Bakterien der Gattung *Erwinia* ist/(sind).

## Claims (Claims for the following Contracting State(s): AT, BE, CH, DE, DK, FR, GB, GR, IE, IT, LI, LU, MC, NL, PT, SE)

1. Procedure for the identification and detection of pectinolytic bacteria of the *Erwinia* genus of a determined species, and more particularly of the species *Erwinia chrysanthemi* and the species *Erwinia carotovora,* in the soil or in water, or in a host capable of being a carrier of such bacteria, in particular in plants and seeds, this procedure consisting of screening the DNA or RNA fragments specific to the species, subspecies or pathovar concerned, coding a pectate-lyase (PL), using a molecular hybridization with at least one specific probe, this hybridization being preceded by an amplification of the number of copies of the DNA fragments mentioned above using specific primers.

2. Nucleotide sequence characterized in that it includes all or part of the following nucleotide sequence A: 5'-TTACCGGACGCCGAGCTGTGGCGT-3' or its complementary sequence, or a sequence derived from sequence A, in particular by the suppression and/or substitution and/or addition of nucleotides, and capable of hybridizing with the fragment delimited by positions 1231 and 1254 of the sequence represented in Figure 1, or the complementary sequence of this derived sequence.

3. Nucleotide sequence characterized in that it contains all or part of nucleotide sequence B chosen from all or part:
* of the concatenations of the following nucleotides: 
5'-CAGGAAGATGTCGTTATCGCGAGT-3'
.........C.............
.........T.............
 or their complementary sequences,
* or of a sequence derived from sequence B, in particular by the suppression and/or substitution and/or addition of nucleotides, and capable of hybridizing with the complementary sequence of the fragment delimited by positions 1642 and 1665 of the sequence represented in Figure 1, or the complementary sequence of this derived sequence.

4. Nucleotide sequence characterized in that it contains all or part of nucleotide sequence C chosen from all or part:
* of the concatenations of the following nucleotides: 
5'-GATCAGAAAGCCCGCAGCCAGAT-3'
.....A........T........
...........G..T........
 (the dots representing nucleotides which are identical to those of the first line) or their complementary sequences,
* or of a sequence derived from sequence C, in particular by the suppression and/or substitution and/or addition of nucleotides, and capable of hybridizing with the fragment delimited by positions 295 and 317 of the sequence represented in Figure 2, or the complementary sequence of this derived sequence.

5. Nucleotide sequence characterized in that it contains all or part of nucleotide sequence D chosen from all or part:
* of the concatenations of the following nucleotides: 
5'-CTGTGGCCGATCAGGATGGTTTTGTCGTGC-3'
..A.......................A...
T.............C...............
.....A..A.....C...............
.................A............
 or their complementary sequences,
* or of a sequence derived from sequence D, in particular by the suppression and/or substitution and/or addition of nucleotides, and capable of hybridizing with the complementary sequence of the fragment delimited by positions 672 and 701 of the sequence represented in Figure 2, or the complementary sequence of this derived sequence.

6. Primer pairs for gene amplification, characterized in that one of the primers of these pairs is chosen from the sequences containing all or part of nucleotide sequence A, or a derived sequence, according to claim 2, while the other primer is chosen from the sequences containing all or part of nucleotide sequence B, or a derived sequence, according to claim 3.

7. Primer pairs for gene amplification, characterized in that one of the primers of these pairs is chosen from the sequences containing all or part of nucleotide sequence C, or a derived sequence, according to claim 4, while the other primer is chosen from the sequences containing all or part of nucleotide sequence D, or a derived sequence according to claim 5.

8. Nucleotide probe characterized in that it contains at least 10 nucleotides of the sequence delimited by the nucleotides situated at positions 1231 and 1665 of the sequence represented in Figure 1, or at least 10 nucleotides having a minimum 40% homology with the sequence mentioned above.

9. Nucleotide probe characterized in that it contains at least 10 nucleotides of the sequence delimited by the nucleotides situated at positions 295 and 701 of the sequence represented in Figure 2, or at least 10 nucleotides having a minimum 40% homology with the sequence mentioned above.

10. Nucleotide sequences according to one of claims 2 to 9, characterized in that they are labelled, in particular in a radioactive, enzymatic manner, by a fluorescent compound, by linkage to an antigenic molecule (capable of being recognised by the antibodies) or any other molecule capable of being directly or indirectly detected using reagents, this linkage being able to be carried out by means of a spacer arm, in particular by means of a nucleotide sequence constituted by about 5 to 100 nucleotides situated at the 3' or 5' ends of these sequences.

11. Identification and detection procedure according to claim 1, for *Erwinia carotovora* in the soil or in water, or in a host, in particular in plants and seeds, this procedure including the following stages:
- treatment of a sample taken from water, the soil, or from a host, in a way so as to render the genome of *Erwinia carotovora* accessible to the primers defined in claim 6, and if appropriate to any DNA or RNA polymerase allowing the replication of the two strands of the genomic DNA, or the RNA deriving from it, this treatment being notably carried out by boiling, maceration (in a liquid such as water), grinding, or sonication,
- amplification of the number of copies of the gene fragments coding the PL's, capable of being present in this sample, using primer pairs as defined above,
- detection of the possible presence of genes coding the PL'S, and therefore the presence of *Erwinia carotovora* in the sample studied, using a probe according to claim 8, if appropriate labelled, in particular in the manner indicated in claim 10.

12. Identification and detection procedure according to claim 1, for *Erwinia chrysanthemi* in the soil or in water, or in a host, in particular in plants and seeds, this procedure including the following stages:
- treatment of a sample taken from water, the soil, or from a host, in a way so as to render the genome of *Erwinia chrysanthemi* accessible to the primers defined in claim 7, and if appropriate to any DNA or RNA polymerase allowing the replication of the two strands of the genomic DNA, or the RNA deriving from it, this treatment being notably carried out by boiling, maceration (in a liquid such as water), grinding, or sonication,
- amplification of the number of copies of the gene fragments coding the PL's, capable of being present in this sample, using primer pairs as defined above,
- detection of the possible presence of genes coding the PL's, and therefore the presence of *Erwinia chrysanthemi* in the sample studied, using a probe according to claim 9, if appropriate labelled, in particular in the manner indicated in claim 10.

13. Procedure according to claim 11 or 12, characterized in that the amplification of the number of genes coding pectate-lyases includes the following stages:
- predenaturation of the double strand DNA into single strand DNA, preferably in a buffer constituted by Tris-HCl 10 mM pH 8.3, 50 mM KCl, 1.5 mM MgCl₂, 0.01% gelatine, co-factors of DNA(or RNA)-polymerase, in particular Mg²⁺ and K⁺ ions, the constituent 4 deoxynucleotides of the DNA's (dCTP, dATP, dGTP, dTTP), or of the RNA's (dCTP, dUTP, dGTP, dTTP) and the primer pairs as defined in claim 6 or 7, by heating at between about 80°C and about 110°C, advantageously at 100°C,
- the actual amplification by addition of for example thermoresistant DNA polymerase to the medium obtained in the previous stage, and
* heating to about 94°C, which corresponds to the actual denaturation stage,
* then heating to between about 60°C and about 76°C, which corresponds to the hybridization stage of the primer pairs with the genes coding the PL's, or fragments of these genes, capable of being present in the biological sample studied,
* and finally heating to between about 50°C and about 76°C, which corresponds to the elongation stage of the primers, hybridized in the previous stage, one to the other, in this way producing complementary nucleotide sequences of all or part of the genomic sequences of pectinolytic erwinias, these last-named sequences being delimited by the nucleotides hybridizing with the primers mentioned above,
- repetition of the previous amplification stage between about 20 and about 50 times, advantageously between 25 and 35 times,
- if appropriate, the recovery of an aliquot part of the medium obtained at the end of the preceding amplification stage in order to carry out a new amplification according to the method described above.

14. Use of the procedure according to one of claims 11 to 13, for the implementation of a diagnostic method for the possible appearance of pathologies caused by the bacteria of the species *Erwinia carotovora* and more particularly by one of the subspecies *Ecc, Eca, Eco* and *Ecw,* by the bacteria of the species *Erwinia chrysanthemi,* or any species, subspecies or pathovar, current or derived subsequently by modification of the current taxonomy.

15. Kit for the implementation of a procedure according to one of claims 11 to 13, characterized in that it contains:
- at least one pair of primers according to claim 6, and/or
- at least one pair of primers according to claim 7,
- one (or more) probe(s) according to claim 8, and/or one (or more) probe(s) according to claim 9, said probes being capable of hybridizing with all or part of the gene fragment amplified by means of the primer pairs according to claims 6 and 7 respectively.

16. Kit according to claim 15, characterized in that it also contains:
- a thermoresistant DNA or RNA polymerase,
- a reaction medium advantageously constituted by Tris-HCl 10 mM pH 8.3, 50 mM KCl, 1.5 mM MgCl₂, 0.01% gelatine, co-factors of DNA polymerase, notably Mg²⁺ and K⁺ ions, and the 4 constitutive deoxynucleotides of DNA (dCTP, dATP, dGTP, dTTP) or RNA (dCTP, dATP, dGTP, dUTP) and/or,
one or more restriction enzymes, and, if appropriate, the reference restriction fragments characteristic of the subspecies of *Erwinia carotovora* and/or *Erwinia chrysanthemi,* in particular the restriction fragments represented in Figure 3, in the case of the subspecies of *Erwinia carotovora* and/or,
- a ligase and one (or more) pair(s) of oligonucleotide sequences, capable of hybridizing on either side of one (or more) nucleotide(s) situated in the genes or gene fragments coding pectate-lyases, and being characteristic of a species, subspecies or pathovar of *Erwinia,* and/or
- one (or more) oligonucleotide probe(s) specific to one nucleotide region characteristic of a determined species, subspecies or pathovar of bacteria of the *Erwinia* genus.

17. Use of the nucleotide sequences corresponding to all or part of the genes which, within the genomes of the pectinolytic bacteria of the *Erwinia* genus, and more particularly of *Erwinia carotovora* and *Erwinia chrysanthemi,* coding pectate-lyases, for the implementation of an identification procedure for subspecies and/or strains or pathovars of species or subspecies of the different bacteria of the *Erwinia* genus.

## Claims (Claims for the following Contracting State(s): ES)

1. Procedure for the identification and detection of pectinolytic bacteria of the *Erwinia* genus of a determined species, and more particularly of the species *Erwinia chrysanthemi* and the species *Erwinia carotovora,* in the soil or in water, or in a host capable of being a carrier of such bacteria, in particular in plants and seeds, this procedure consisting of screening the DNA or RNA fragments specific to the species, subspecies or pathovar concerned, coding a pectate-lyase (PL), using a molecular hybridization with at least one specific probe, this hybridization being preceded by an amplification of the number of copies of the DNA fragments mentioned above using specific primers.

2. Nucleotide sequence characterized in that it includes all or part of the following nucleotide sequence A: 5'-TTACCGGACGCCGAGCTGTGGCGT-3' or its complementary sequence, or a sequence derived from sequence A, in particular by the suppression and/or substitution and/or addition of nucleotides, and capable of hybridizing with the fragment delimited by positions 1231 and 1254 of the sequence represented in Figure 1, or the complementary sequence of this derived sequence.

3. Nucleotide sequence characterized in that it contains all or part of nucleotide sequence B chosen from all or part:
* of the concatenations of the following nucleotides: 
5'-CAGGAAGATGTCGTTATCGCGAGT-3'
.........C..............
.........T..............
 or their complementary sequences,
* or of a sequence derived from sequence B, in particular by the suppression and/or substitution and/or addition of nucleotides, and capable of hybridizing with the complementary sequence of the fragment delimited by positions 1642 and 1665 of the sequence represented in Figure 1, or the complementary sequence of this derived sequence.

4. Nucleotide sequence characterized in that it contains all or part of nucleotide sequence C chosen from all or part:
* of the concatenations of the following nucleotides: 
5'-GATCAGAAAGCCCGCAGCCAGAT-3'
.....A........T........
...........G..T........
 (the dots representing nucleotides which are identical to those of the first line) or their complementary sequences,
* or of a sequence derived from sequence C, in particular by the suppression and/or substitution and/or addition of nucleotides, and capable of hybridizing with the fragment delimited by positions 295 and 317 of the sequence represented in Figure 2, or the complementary sequence of this derived sequence.

5. Nucleotide sequence characterized in that it contains all or part of nucleotide sequence D chosen from all or part:
* of the concatenations of the following nucleotides: 
5'-CTGTGGCCGATCAGGATGGTTTTGTCGTGC-3'
..A.......................A...
T.............C...............
.....A..A.....C...............
.................A............
 or their complementary sequences,
* or of a sequence derived from sequence D, in particular by the suppression and/or substitution and/or addition of nucleotides, and capable of hybridizing with the complementary sequence of the fragment delimited by positions 672 and 701 of the sequence represented in Figure 2, or the complementary sequence of this derived sequence.

6. Primer pairs for gene amplification, characterized in that one of the primers of these pairs is chosen from the sequences containing all or part of nucleotide sequence A, or a derived sequence, according to claim 2, while the other primer is chosen from the sequences containing all or part of nucleotide sequence B, or a derived sequence, according to claim 3.

7. Primer pairs for gene amplification, characterized in that one of the primers of these pairs is chosen from the sequences containing all or part of nucleotide sequence C, or a derived sequence, according to claim 4, while the other primer is chosen from the sequences containing all or part of nucleotide sequence D, or a derived sequence according to claim 5.

8. Nucleotide probe characterized in that it contains at least 10 nucleotides of the sequence delimited by the nucleotides situated at positions 1231 and 1665 of the sequence represented in Figure 1, or at least 10 nucleotides having a minimum 40% homology with the sequence mentioned above.

9. Nucleotide probe characterized in that it contains at least 10 nucleotides of the sequence delimited by the nucleotides situated at positions 295 and 701 of the sequence represented in Figure 2, or at least 10 nucleotides having a minimum 40% homology with the sequence mentioned above.

10. Nucleotide sequences according to one of claims 2 to 9, characterized in that they are labelled, in particular in a radioactive, enzymatic manner, by a fluorescent compound, by linkage to an antigenic molecule (capable of being recognised by the antibodies) or any other molecule capable of being directly or indirectly detected using reagents, this linkage being able to be carried out by means of a spacer arm, in particular by means of a nucleotide sequence constituted by about 5 to 100 nucleotides situated at the 3' or 5' ends of these sequences.

11. Identification and detection procedure according to claim 1, for *Erwinia carotovora* in the soil or in water, or in a host, in particular in plants and seeds, this procedure including the following stages:
- treatment of a sample taken from water, the soil, or from a host, in a way so as to render the genome of *Erwinia carotovora* accessible to the primers defined in claim 6, and if appropriate to any DNA or RNA polymerase allowing the replication of the two strands of the genomic DNA, or the RNA deriving from it, this treatment being notably carried out by boiling, maceration (in a liquid such as water), grinding, or sonication,
- amplification of the number of copies of the gene fragments coding the PL's, capable of being present in this sample, using primer pairs as defined above,
- detection of the possible presence of genes coding the PL'S, and therefore the presence of *Erwinia carotovora* in the sample studied, using a probe according to claim 8, if appropriate labelled, in particular in the manner indicated in claim 10.

12. Identification and detection procedure according to claim 1, for *Erwinia chrysanthemi* in the soil or in water, or in a host, in particular in plants and seeds, this procedure including the following stages:
- treatment of a sample taken from water, the soil, or from a host, in a way so as to render the genome of *Erwinia chrysanthemi* accessible to the primers defined in claim 7, and if appropriate to any DNA or RNA polymerase allowing the replication of the two strands of the genomic DNA, or the RNA deriving from it, this treatment being notably carried out by boiling, maceration (in a liquid such as water), grinding, or sonication,
- amplification of the number of copies of the gene fragments coding the PL's, capable of being present in this sample, using primer pairs as defined above,
- detection of the possible presence of genes coding the PL's, and therefore the presence of *Erwinia chrysanthemi* in the sample studied, using a probe according to claim 9, if appropriate labelled, in particular in the manner indicated in claim 10.

13. Procedure according to claim 11 or 12, characterized in that the amplification of the number of genes coding pectate-lyases includes the following stages:
- predenaturation of the double strand DNA into single strand DNA, preferably in a buffer constituted by Tris-HCl 10 mM pH 8.3, 50 mM KCl, 1.5 mM MgCl₂, 0.01% gelatine, co-factors of DNA(or RNA)-polymerase, in particular Mg²⁺ and K⁺ ions, the constituent 4 deoxynucleotides of the DNA's (dCTP, dATP, dGTP, dTTP), or of the RNA's (dCTP, dUTP, dGTP, dTTP) and the primer pairs as defined in claim 6 or 7, by heating at between about 80°C and about 110°C, advantageously at 100°C,
- the actual amplification by addition of for example thermoresistant DNA polymerase to the medium obtained in the previous stage, and
* heating to about 94°C, which corresponds to the actual denaturation stage,
* then heating to between about 60°C and about 76°C, which corresponds to the hybridization stage of the primer pairs with the genes coding the PL's, or fragments of these genes, capable of being present in the biological sample studied,
* and finally heating to between about 50°C and about 76°C, which corresponds to the elongation stage of the primers, hybridized in the previous stage, one to the other, in this way producing complementary nucleotide sequences of all or part of the genomic sequences of pectinolytic erwinias, these last-named sequences being delimited by the nucleotides hybridizing with the primers mentioned above,
- repetition of the previous amplification stage between about 20 and about 50 times, advantageously between 25 and 35 times,
- if appropriate, the recovery of an aliquot part of the medium obtained at the end of the preceding amplification stage in order to carry out a new amplification according to the method described above.

14. Use of the procedure according to one of claims 11 to 13, for the implementation of a diagnostic method for the possible appearance of pathologies caused by the bacteria of the species *Erwinia carotovora* and more particularly by one of the subspecies *Ecc, Eca, Eco* and *Ecw,* by the bacteria of the species *Erwinia chrysanthemi,* or any species, subspecies or pathovar, current or derived subsequently by modification of the current taxonomy.

15. Kit for the implementation of a procedure according to one of claims 11 to 13, characterized in that it contains:
- at least one pair of primers according to claim 6, and/or
- at least one pair of primers according to claim 7,
- one (or more) probe(s) according to claim 8, and/or one (or more) probe(s) according to claim 9, said probes being capable of hybridizing with all or part of the gene fragment amplified by means of the primer pairs according to claims 6 and 7 respectively.

16. Kit according to claim 15, characterized in that it also contains:
- a thermoresistant DNA or RNA polymerase,
- a reaction medium advantageously constituted by Tris-HCl 10 mM pH 8.3, 50 mM KCl, 1.5 mM MgCl₂, 0.01% gelatine, co-factors of DNA polymerase, notably Mg²⁺ and K⁺ ions, and the 4 constitutive deoxynucleotides of DNA (dCTP, dATP, dGTP, dTTP) or RNA (dCTP, dATP, dGTP, dUTP) and/or,
one or more restriction enzymes, and, if appropriate, the reference restriction fragments characteristic of the subspecies of *Erwinia carotovora* and/or *Erwinia chrysanthemi,* in particular the restriction fragments represented in Figure 3, in the case of the subspecies of *Erwinia carotovora* and/or,
- a ligase and one (or more) pair(s) of oligonucleotide sequences, capable of hybridizing on either side of one (or more) nucleotide(s) situated in the genes or gene fragments coding pectate-lyases, and being characteristic of a species, subspecies or pathovar of *Erwinia,* and/or
- one (or more) oligonucleotide probe(s) specific to one nucleotide region characteristic of a determined species, subspecies or pathovar of bacteria of the *Erwinia* genus.

17. Use of the nucleotide sequences corresponding to all or part of the genes which, within the genomes of the pectinolytic bacteria of the *Erwinia* genus, and more particularly of *Erwinia carotovora* and *Erwinia chrysanthemi,* coding pectate-lyases, for the implementation of an identification procedure for subspecies and/or strains or pathovars of species or subspecies of the different bacteria of the *Erwinia* genus.

18. Process for preparing a nucleotide sequence comprising all or part of the following nucleotide sequence A: 5'-TTACCGGACGCCGAGCTGTGGCGT-3' or its complementary sequence, or a sequence derived from sequence A, in particular by the suppression and/or substitution and/or addition of nucleotides, and capable of hybridizing with the fragment delimited by positions 1231 and 1254 of the sequence represented in Figure 1, or the complementary sequence of this derived sequence, said process being carried out either by biological route, in particular after transformation and cloning of cellular hosts capable of containing this nucleotide sequence, or amplification of the number of copies of this nucleotide sequence, in particular according to the PCR technique, or by chemical route, in particular by the automated β-cyanoethyl phosphoramidite method described in Bioorganic Chemistry 4; 274-325, 1986, or by the assembling method, for example by abutment (or ligation, by the action of a ligase), of chemically synthesized oligonucleotides, provided at their ends with adequate restriction sites, according to the principle described in Proc. Nat. Acad. Sci. USA, 80; 7461-7465, 1983, these methods being followed by the cloning of the DNA's thus obtained in an appropriate vector, in particular a plasmid vector, and the recovery of the DNA's by hybridization with an appropriate probe, or by electrophoresis, generally after restriction, followed by an elution of the fragments.

19. Process for preparing a nucleotide sequence comprising all or part of nucleotide sequence B chosen from all or part:
* of the concatenations of the following nucleotides: 
5'-CAGGAAGATGTCGTTATCGCGAGT-3'
.........C..............
.........T..............
 or their complementary sequences,
* or of a sequence derived from sequence B, in particular by the suppression and/or substitution and/or addition of nucleotides, and capable of hybridizing with the complementary sequence of the fragment delimited by positions 1642 and 1665 of the sequence represented in Figure 1, or the complementary sequence of this derived sequence, said process being carried out according to methods described in claim 18.

20. Process for preparing a nucleotide sequence comprising all or part of nucleotide sequence C chosen from all or part:
* of the concatenations of the following nucleotides: 
5'-GATCAGAAAGCCCGCAGCCAGAT-3'
.....A........T........
...........G..T........
 (the dots representing nucleotides which are identical to those of the first line) or their complementary sequences,
* or of a sequence derived from sequence C, in particular by the suppression and/or substitution and/or addition of nucleotides, and capable of hybridizing with the fragment delimited by positions 295 and 317 of the sequence represented in Figure 2, or the complementary sequence of this derived sequence, said process being carried out according to methods described in claim 18.

21. Process for preparing a nucleotide sequence comprising all or part of nucleotide sequence D chosen from all or part:
* of the concatenations of the following nucleotides: 
5'-CTGTGGCCGATCAGGATGGTTTTGTCGTGC-3'
..A.......................A...
T.............C...............
.....A..A.....C...............
.................A............
 or their complementary sequences,
* or of a sequence derived from sequence D, in particular by the suppression and/or substitution and/or addition of nucleotides, and capable of hybridizing with the complementary sequence of the fragment delimited by positions 672 and 701 of the sequence represented in Figure 2, or the complementary sequence of this derived sequence, said process being carried out according to methods described in claim 18.

22. Process for preparing primer pairs for gene amplification, one of the primers of these pairs being chosen from the sequences containing all or part of nucleotide sequence A, or a derived sequence, according to claim 18, while the other primer is chosen from the sequences containing all or part of nucleotide sequence B, or a derived sequence, according to claim 19, said process being carried out according to methods described in claim 18.

23. Process for preparing primer pairs for gene amplification, one of the primers of these pairs being chosen from the sequences containing all or part of nucleotide sequence C, or a derived sequence, according to claim 20, while the other primer is chosen from the sequences containing all or part of nucleotide sequence D, or a derived sequence according to claim 21, said process being carried out according to methods described in claim 18.

24. Process for preparing a nucleotide probe comprising at least 10 nucleotides of the sequence delimited by the nucleotides situated at positions 1231 and 1665 of the sequence represented in Figure 1, or at least 10 nucleotides having a minimum 40% homology with the sequence mentioned above, said process being carried out according to methods described in claim 18.

25. Process for preparing a nucleotide probe comprising at least 10 nucleotides of the sequence delimited by the nucleotides situated at positions 295 and 701 of the sequence represented in Figure 2, or at least 10 nucleotides having a minimum 40% homology with the sequence mentioned above, said process being carried out according to methods described in claim 18.

26. Process for preparing nucleotide sequences as defined in one of claims 18 to 25, labelled, in particular in a radioactive, enzymatic manner, by a fluorescent compound, by linkage to an antigenic molecule (capable of being recognised by the antibodies) or any other molecule capable of being directly or indirectly detected using reagents, this linkage being able to be carried out by means of a spacer arm, in particular by means of a nucleotide sequence constituted by about 5 to 100 nucleotides situated at the 3' or 5' ends of these sequences, said process being carried out according to methods described in claim 18.

27. Process for preparing a kit for the implementation of a procedure according to one of claims 11 to 13, said process comprising the step consisting in gathering :
- at least one pair of primers according to claim 22, and/or
- at least one pair of primers according to claim 23,
- one (or more) probe(s) according to claim 24, and/or one (or more) probe(s) according to claim 25, said probes being capable of hybridizing with all or part of the gene fragment amplified by means of the primer pairs according to claims 22 and 23 respectively.

28. Process for preparing a kit according to claim 27, said process comprising also the step consisting in gathering :
- a thermoresistant DNA or RNA polymerase,
- a reaction medium advantageously constituted by Tris-HCl 10 mM pH 8.3, 50 mM KCl, 1.5 mM MgCl₂, 0.01% gelatine, co-factors of DNA polymerase, notably Mg²⁺ and K⁺ ions, and the 4 constitutive deoxynucleotides of DNA (dCTP, dATP, dGTP, dTTP) or RNA (dCTP, dATP, dGTP, dUTP) and/or,
one or more restriction enzymes, and, if appropriate, the reference restriction fragments characteristic of the subspecies of *Erwinia carotovora* and/or *Erwinia chrysanthemi,* in particular the restriction fragments represented in Figure 3, in the case of the subspecies of *Erwinia carotovora* and/or,
- a ligase and one (or more) pair(s) of oligonucleotide sequences, capable of hybridizing on either side of one (or more) nucleotide(s) situated in the genes or gene fragments coding pectate-lyases, and being characteristic of a species, subspecies or pathovar of *Erwinia,* and/or
- one (or more) oligonucleotide probe(s) specific to one nucleotide region characteristic of a determined species, subspecies or pathovar of bacteria of the *Erwinia* genus.
